# EUROPEAN PATENT SPECIFICATION

(11) **EP 4 069 243 B1**
(45) Date of publication and mention of the grant of the patent: **01.04.2026**
(21) Application number: 21738244.9
(22) Date of filing: 08.01.2021
(51) Int. Cl.: A61K 31/522, A61K 31/502, A61K 45/06, A61P 35/00

(54) **COMBINATIONS OF ETC-159 AND OLAPARIB FOR USE IN THE TREATMENT OF PANCREATIC CANCER, OVARIAN CANCER AND CHOLANGIO-CARCINOMAS**
KOMBINATIONEN VON ETC-159 UND OLAPARIB ZUR BEHANDLUNG VON BAUCHSPEICHELDRÜSENKREBS, EIERSTOCKKREBS AND CHOLANGIOKARZINOM
COMBINAISONS D'ETC-159 ET D'OLAPARIB POUR LE TRAITEMENT DU CANCER DU PANCRÉAS, DU CANCER DE L'OVAIRE ET DU CHOLANGIOCARCINOME

(30) Priority: 08.01.2020 SG 10202000183R
(43) Date of publication of application: 12.10.2022
(73) Proprietor: Agency for Science, Technology and Research, Singapore 138632 (SG); National University of Singapore, Singapore 119077 (SG)
(72) Inventor: LEE, May Ann, Singapore 138670 (SG); D/O SEPRAMANIAM, Sugunavathi, Singapore 138670 (SG); M VIRSHUP, David Marc, Singapore 119077 (SG); MADAN, Babita, Singapore 119077 (SG)
(74) Representative: Appleyard Lees IP LLP
(86) International application number: PCT/SG2021/050009
(87) International publication number: WO 2021/141538

(56) References cited:
- WO-A1-2014/189466
- ZHONG ZHENG ET AL: "PORCN inhibition synergizes with PI3K/mTOR inhibition in Wnt-addicted cancers", ONCOGENE, NATURE PUBLISHING GROUP UK, LONDON, vol. 38, no. 40, 7 August 2019 (2019-08-07), pages 6662 - 6677, XP036897095, ISSN: 0950-9232, [retrieved on 20190807], DOI: 10.1038/S41388-019-0908-1
- ZHONG ZHENG ET AL: "Wnts and the hallmarks of cancer", CANCER METASTASIS, KLUWER ACADEMIC PUBLISHERS, DORDRECHT, NL, vol. 39, no. 3, 8 May 2020 (2020-05-08), pages 625 - 645, XP037243192, ISSN: 0167-7659, [retrieved on 20200508], DOI: 10.1007/S10555-020-09887-6
- YAMAMOTO TOMOMI M., MCMELLEN ALEXANDRA, WATSON ZACHARY L., AGUILERA JENNIFER, FERGUSON REBECCA, NURMEMMEDOV ELMAR, THAKAR TANAY, M: "Activation of Wnt signaling promotes olaparib resistant ovarian cancer", MOLECULAR CARCINOGENESIS, vol. 58, no. 10, 1 October 2019 (2019-10-01), US , pages 1770 - 1782, XP055940529, ISSN: 0899-1987, DOI: 10.1002/mc.23064
- MADAN, B. ET AL.: "Wnt addiction of genetically defined cancers reversed by PORCN inhibition", ONCOGENE, vol. 35, 10 August 2015 (2015-08-10), pages 2197 - 2207, XP055496939, [retrieved on 20210301], DOI: 10.1038/ONC.2015.280
- KAUR AMANPREET, LIM JUN YI STANLEY, SEPRAMANIAM SUGUNAVATHI, PATNAIK SIDDHI, HARMSTON NATHAN, LEE MAY ANN, PETRETTO ENRICO, VIRSHU: "WNT inhibition creates a BRCA‐like state in Wnt‐addicted cancer", EMBO MOLECULAR MEDICINE, WILEY-BLACKWELL, vol. 13, no. 4, 9 April 2021 (2021-04-09), XP055840746, ISSN: 1757-4676, DOI: 10.15252/emmm.202013349

## Description

### Field of the Invention

The present invention relates to a synergistic combination of ETC-159 and olaparib for use in the treatment of certain cancers.

### Background of the Invention

Cancer is typically treated with either chemotherapy and/or radiation therapy. While often effective to destroy a significant amount of tumour cells, such therapies often leave behind a number of tumour cells that are resistant to the treatment. These resistant cells can proliferate to form new tumors that are then resistant to treatment. The use of known combinations of chemotherapeutic drugs has on occasion given rise to multidrug resistant ('MDR') tumour cells.

Further, many diseases including cancer are driven by complex molecular interactions that dysregulate several pathways at any one time. Hence therapeutically targeting a single component in one of the pathways may not be sufficient to disrupt or rectify these complex mechanisms. Due to the scale of complexity, early drug discovery studies have increasingly evolved to target multiple molecules, pathways, or networks. In the treatment of such diseases, drugs with different mechanisms may be combined (i.e, combination therapies) with beneficial effects including the effective treatment of MDR tumour cells and the minimisation of side effects such as undesireable cytotoxicity. Therapeutic approaches using combinations of drugs directed at multiple targets can improve treatment response, provide better efficacy, decreased toxicity or minimize development of resistance. However, recognising efficacious combinations is not straightforward as many drugs combinations are incompatible with each other, especially in the oncology setting. For example, Figitumumab (Pfizer) was developed to inhibit a specific growth factor (IGF-1R) for the treatment of lung cancer and it showed positive results in animal testing. However, in phase 3 trials, combining Figitumumab with established DNA intercalating chemotherapy regimens (specifically carboplatin and paclitaxel) failed to improve survival, and in combination with one regimen increased serious adverse events and deaths to 21% compared to the 12% when using carboplatin or paclitaxel chemotherapy alone. In this regard, determining which combination of pathways to select to target a disease represents a challenge.

While drug combinations are theoretically feasible, on a practical level, the effects of drug combinations are unpredictable. The effect of co-administrating multiple drugs is often described as synergistic, additive or antagonistic. This illustrates that not all drug combinations provide useful or beneficial effects. It is not predictable from the outset that specific combinations will provide a synergistic effect. In some cases, the combination may even amplify the adverse side effects, which is not desirable. For example, interaction of warfarin with tamoxifen, or with capecitabine and paclitaxel can enhance the risk of bleeding and/or haemorrhage. In another example, a combination of NSAID and bisphosphonates can result in an amplified risk of gastric ulcers.

Wnts are a family of 19 evolutionarily conserved cysteine rich morphogens that interact with at least 15 different receptors and co-receptors to regulate a multitude of developmental and homeostatic processes, such as cell proliferation, cell polarity and cell fate determination during embryonic development and tissue homeostasis. Wnts signal through both β-catenin-dependent and β-catenin-independent pathways. Three Wnt signaling pathways have been characterized: the canonical Wnt pathway, the noncanonical planar cell polarity pathway, and the noncanonical Wnt/calcium pathway. Dysregulation of Wnt signaling may result in a subset of cancers due to mutations in either upstream or downstream components. Wnt signalling is one of the key oncogenic pathways in multiple cancers, and accordingly, targeting this pathway is an attractive therapeutic approach.

Oncogene 2015, 35, 2197 discloses that Wnt addiction of genetically defined cancers reversed by PORCN inhibition. ETC-159 is demonstrated to block the secretion of all Wnts and the article concludes that the inhibition of Wnt signalling by PORCN inhibition holds promise as differentiation therapy in genetically defined human cancers.

WO 2014/189466 A1 discloses purine-diones, including ETC-159, as Wnt pathway modulators for use in the treatment of cancer.

Accordingly, a research need exists to develop new and useful anti-proliferative combinations.

It is generally desirable to overcome or ameliorate one or more of the above described difficulties.

### Summary of the Invention

The invention is defined by the claims. Any subject-matter falling outside the scope of the claims is provided for information purposes only. Any references in the description to methods of treatment, diagnosis or surgery refer to the compounds, pharmaceutical compositions and medicaments of the present invention for use in a method for treatment, diagnosis or surgery of the human or animal body by therapy.

The present invention provides a pharmaceutical combination for use in treating pancreatic cancer, ovarian cancer, or cholangio-carcinomas comprising (a) a compound of formula (I) or a pharmaceutically acceptable salt, or solvate thereof 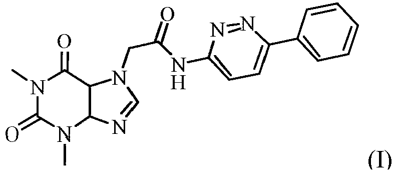 and (b) Olaparib.

The present invention further provides a combination wherein (a) a compound of formula (I) or a pharmaceutically acceptable salt, or solvate thereof and (b) Olaparib are for administration simultaneously or sequentially in any order.

The present invention provides a pharmaceutical composition comprising (a) a compound of formula (I) or a pharmaceutically acceptable salt, or solvate thereof and (b) Olaparib.

Olaparib (AZD-2281; trade name Lynparza) developed by KuDOS Pharmaceuticals and AstraZeneca, is shown below as the compound of formula (II) and in the context of the present invention encompasses its pharmaceutically acceptable salts, or solvates thereof

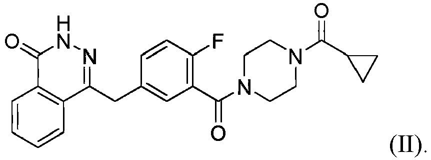

In an embodiment, the cancer is characterised by abnormal, optionally high Wnt activity. Examples of such cancer, may be but not limited to, cervical, colon, breast, bladder, head and neck, gastric, lung, ovarian, prostate, thyroid, non-small-cell lung, mesothelioma, melanoma, pancreatic adenocarcinoma, basal cell carcinoma, osteosarcoma, hepatocellular carcinoma, Wilm's tumor, medulloblastoma or cholangio-carcinoma.

Unexpectedly, it has been found that a synergistic effect may be achieved in the tretment of certain cancers using a combination of a compound of Formula (I) and olaparib.

### Brief Description of the Figures

**Figure 1** is an illustration of a dose-response assay plate for testing compounds at different concentrations based on the IC₅₀ values of each compound.
**Figure 2** is an illustration of a dose-response assay plate containing EGI-1 cells (Test 1) after testing compounds (alone and in combination) at different concentrations based on the IC₅₀ values of each compound.
**Figure 3** is an illustration of a dose-response assay plate containing EGI-1 cells (Test 2) after testing compounds (alone and in combination) at different concentrations based on the IC₅₀ values of each compound.
**Figure 4** is an illustration of a dose-response assay plate containing HPAF-II cells (Test 1) after testing compounds (alone and in combination) at different concentrations based on the IC₅₀ values of each compound.
**Figure 5** is an illustration of a dose-response assay plate containing HPAF-II cells (Test 2) after testing compounds (alone and in combination) at different concentrations based on the IC₅₀ values of each compound.
**Figure 6** is an illustration of a dose-response assay plate containing MCAS cells (Test 1) after testing compounds (alone and in combination) at different concentrations based on the IC₅₀ values of each compound.
**Figure 7** is an illustration of a dose-response assay plate containing MCAS cells (Test 2) after testing compounds (alone and in combination) at different concentrations based on the IC₅₀ values of each compound.
**Figure 8** is an illustration of a dose-response assay plate containing CFPAC-1 cells (Test 1) after testing compounds (alone and in combination) at different concentrations based on the IC₅₀ values of each compound.
**Figure 9** is an illustration of a dose-response assay plate containing CFPAC-1 cells (Test 2) after testing compounds (alone and in combination) at different concentrations based on the IC₅₀ values of each compound.
**Figure 10** is an illustration of a dose-response assay plate containing TFK-1 cells (Test 1) after testing compounds (alone and in combination) at different concentrations based on the IC₅₀ values of each compound.
**Figure 11** is an illustration of a dose-response assay plate containing TFK-1 cells (Test 2) after testing compounds (alone and in combination) at different concentrations based on the IC₅₀ values of each compound.
**Figure 12** is an illustration of a dose-response assay plate containing PA-TU-8988T cells (Test 1) after testing compounds (alone and in combination) at different concentrations based on the IC₅₀ values of each compound.
**Figure 13** is an illustration of a dose-response assay plate containing PA-TU-8988T cells (Test 2) after testing compounds (alone and in combination) at different concentrations based on the IC₅₀ values of each compound.

### Detailed Description of the Invention

Throughout this specification and the statements which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, "IC₅₀" is the half maximal inhibitory concentration of a substance where the response (or binding) is reduced by half. Accordingly, IC₅₀ is a measure of the effectiveness of a substance in inhibiting a specific biological or biochemical function. This quantitative measure indicates how much of a substance is needed to inhibit a given biological process (or component of a process, i.e. an enzyme, cell, cell receptor or microorganism) by half. The substance may be an inhibitor. The values are typically expressed as molar concentration. One method of determining the IC₅₀ of a substance is by constructing a dose-response curve and examining the effect of different concentrations of antagonist on reversing agonist activity. IC₅₀ values can be calculated for a given antagonist by determining the concentration needed to inhibit half of the maximum biological response of the agonist. IC₅₀ values can be used to compare the potency of two antagonists. IC₅₀ values are very dependent on conditions under which they are measured. In general, the higher the concentration of inhibitor, the more agonist activity will be lowered. IC₅₀ value increases as agonist concentration increases. Furthermore, depending on the type of inhibition other factors may influence IC₅₀ value. Such are known in the art.

As used herein, "EC₅₀" refers to the concentration of a substance where a response halfway between the baseline and maximum is induced. It is commonly used as a measure of a drug's potency. The EC₅₀ of a *graded* dose response curve therefore represents the concentration of a substance where 50% of its maximal effect is observed. The EC₅₀ of a *quantal* dose response curve represents the concentration of a compound where 50% of the population exhibit a response, after a specified exposure duration. EC₅₀ is related to IC₅₀ which is a measure of a compound's inhibition (50% inhibition). As is known in the art, for competition binding assays and functional antagonist assays IC₅₀ is the most common summary measure of the dose-response curve. For agonist/stimulator assays the most common summary measure is the EC₅₀.

As used herein, a "modulator" is a molecule that can act as an inhibitor or an activator.

As used herein, an "inhibitor" is a molecule that binds to a substrate and decreases its activity. Such a substrate may be an enzyme. Blocking a substrate's activity can kill a pathogen or correct a metabolic imbalance. The binding of an inhibitor can stop another molecule (biomolecule) from entering the substrate's active site and/or hinder the substrate from catalyzing its reaction. Inhibitor binding is either reversible or irreversible. Irreversible inhibitors usually react with the substrate and change it chemically (e.g. via covalent bond formation). These inhibitors modify key amino acid residues needed for enzymatic activity. In contrast, reversible inhibitors bind non-covalently and different types of inhibition are produced depending on the binding and complexation. For example the inhibition may be competitive, uncompetitive, non-competitive or mixed.

As used herein, "inhibit" refers to an act of decreasing a substrate's activity as described above. This action may be performed by a molecule which may be an inhibitor.

As used herein, an "activator" is a molecule that binds to a substrate and increases its activity. They are the opposite of inhibitors.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

### Cancer

As used herein the term "cancer" refers to a disease in which cells are growing and increasing in number uncontrollably. The term broadly encompasses any neoplastic disease including those which are potentially malignant (pre-cancerous) or malignant (cancerous). The term therefore encompasses the treatment of tumours.

Accordingly, the term "tumour" as used herein refers to solid carcinomas and non-solid tumours. Examples of carcinomas are melanomas, colon, lung, ovarian, skin, breast, pancreas, pharynx, brain prostate, CNS, and renal cancers. An example of non-solid tumour is hematological leukemias.

In certain embodiments, the combination may be used in the treatment of a cancer characterised by abnormal, optionally high Wnt activity. Examples of such cancer, may be but are not limited to, cervical, colon, breast, bladder, head and neck, gastric, lung, ovarian, prostate, thyroid, non-small-cell lung, mesothelioma, melanoma, pancreatic adenocarcinoma, basal cell carcinoma, osteosarcoma, hepatocellular carcinoma, Wilm's tumor, medulloblastoma or cholangio-carcinoma.

In its broadest scope, the invention provides a combination of (a) and (b) for use in the treatment of ovarian, pancreatic adenocarcinoma or cholangio-carcinoma.

### Combination partner (a): Wnt modulator

As used herein the term "Wnt modulator" refers to any and all compounds which can either enhance or inhibit Wnt signalling. Therefore, in the present context, a Wnt modulator can either act as an activator or an inhibitor, to either increase or decrease the activity of the Wnt pathway, respectively. In certain embodiments, the Wnt modulator is a Wnt inhibitor. The invention relates specifically to a combination comprising a compound of formula (I) or a pharmaceutically acceptable salt, or solvate thereof:

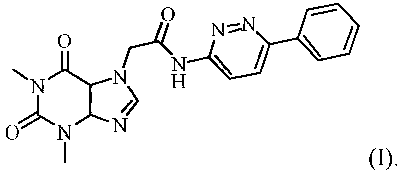

Wnts are a family of 19 evolutionarily conserved cysteine rich morphogens that interact with at least 15 different receptors and co-receptors to regulate a multitude of developmental and homeostatic processes. Wnts signal through both β-catenin-dependent and β-catenin-independent pathways. Three Wnt signaling pathways have been characterized: the canonical Wnt pathway, the noncanonical planar cell polarity pathway, and the noncanonical Wnt/calcium pathway. Dysregulation of Wnt signaling may result in a subset of cancers due to mutations in either upstream or downstream components.

Wnt signalling is one of the key oncogenic pathways in multiple cancers, and targeting this pathway is an attractive therapeutic approach. Nevertheless, aberrations that occur during cancer development and progression are hardly limited to a single pathway even within a given cancer type.

The biochemistry of cancer stem cells is subtly different than that of other tumor cells. These so-called Wnt-addicted cells hijack and depend on constant stimulation of the Wnt pathway to promote their uncontrolled growth, survival and migration. In cancer, it is believed that Wnt signaling can become independent of regular stimuli, through mutations in downstream oncogenes and tumor suppressor genes that become permanently activated even though the normal receptor has not received a signal.

Canonical Wnt pathway activity is believed to be involved in the development of benign and malignant breast tumors. Its presence is revealed by elevated levels of β-catenin in the nucleus and/or cytoplasm, which can be detected with immunohistochemical staining and Western blotting. Increased β-catenin expression is correlated with poor prognosis in breast cancer patients. This accumulation may be due to factors such as mutations in β-catenin, deficiencies in the β-catenin destruction complex, most frequently by mutations in structurally disordered regions of adenomatous polyposis coli (APC), overexpression of Wnt ligands, loss of inhibitors and/or decreased activity of regulatory pathways (such as the Wnt/calcium pathway). Breast tumors can metastasize due to Wnt involvement in epithelial mesenchymal transition (EMT).

Wnt signaling has been implicated in the development of other cancers. Changes in CTNNB 1 expression, which is the gene that encodes β-catenin, can be measured in breast, colorectal, melanoma, prostate, lung, and other cancers. Increased expression of Wnt ligand-proteins such as Wnt 1, Wnt2 and Wnt7A was observed in the development of glioblastoma, oesophageal cancer and ovarian cancer, respectively. Other proteins that cause multiple cancer types in the absence of proper functioning include ROR1, ROR2, SFRP4, Wnt5A, WIF1 and those of the TCF/LEF family.

Without wanting to be bound by theory, it is belived that the compound of formula (I) can treat Wnt-driven proliferative diseases by targeting the level of secretion of Wnts via inhibiting the enzymatic activity of Porcupine (PORCN), an endoplasmic reticulum resident enzyme that post-translationally palmitoleates Wnts at a highly conserved serine residue. PORCN is a membrane-bound O-acyltansferase that palmitoleates Wnts, a process which is essential for Wnt secretion and function. It is believed that this palmitoleation of Wnts is essential for their secretion and binding to the Frizzled receptors. Inhibiting the PORCN enzymatic activity offers an advantage of overcoming the limitations of β-catenin inhibitors that can only block the canonical Wnt signalling pathway. Another advantage is overcoming the anti-Frizzled antibodies that are limited in their ability to target all the Frizzled receptors.

The inventors have further found that the inhibition of Wnt signaling using a compound of Formula (I) also reduces expression of DNA repair genes.

In some embodiments, compound of formula (I) may be administered at about 0.25 times of its IC₅₀ value. In other embodiments, compound of formula (I) may be administered at about 0.5 times of its IC₅₀ value. In other embodiments, compound of formula (I) may be administered at about 0.75 times of its IC₅₀ value. In other embodiments, compound of formula (I) may be administered at about its IC₅₀ value. In other embodiments, compound of formula (I) may be administered at about 1.25 times of its IC₅₀ value. In other embodiments, compound of formula (I) may be administered at about 1.5 times of its IC₅₀ value. In other embodiments, compound of formula (I) may be administered at about 1.75 times of its IC₅₀ value. In other embodiments, compound of formula (I) may be administered at about 2 times of its IC₅₀ value. In other embodiments, compound of formula (I) may be administered at about 3 times of its IC₅₀ value. In other embodiments, compound of formula (I) may be administered at about 4 times of its IC₅₀ value. In other embodiments, compound of formula (I) may be administered at about 5 times of its IC₅₀ value.

In some embodiments, compound of formula (I) may be administered at about 0.25 times of its EC₅₀ value. In other embodiments, compound of formula (I) may be administered at about 0.5 times of its EC₅₀ value. In other embodiments, compound of formula (I) may be administered at about 0.75 times of its EC₅₀ value. In other embodiments, compound of formula (I) may be administered at about its EC₅₀ value. In other embodiments, compound of formula (I) may be administered at about 1.25 times of its EC₅₀ value. In other embodiments, compound of formula (I) may be administered at about 1.5 times of its EC₅₀ value. In other embodiments, compound of formula (I) may be administered at about 1.75 times of its EC₅₀ value. In other embodiments, compound of formula (I) may be administered at about 2 times of its EC₅₀ value. In other embodiments, compound of formula (I) may be administered at about 3 times of its EC₅₀ value. In other embodiments, compound of formula (I) may be administered at about 4 times of its EC₅₀ value. In other embodiments, compound of formula (I) may be administered at about 5 times of its EC₅₀ value.

In some embodiments, compound of formula (I) may be administered at about 0.001 µM. In other embodiments, compound of formula (I) may be administered at about 0.002 µM. In other embodiments, compound of formula (I) may be administered at about 0.005 µM. In other embodiments, compound of formula (I) may be administered at about 0.007 µM. In other embodiments, compound of formula (I) may be administered at about 0.008 µM. In other embodiments, compound of formula (I) may be administered at about 0.01 µM. In other embodiments, compound of formula (I) may be administered at about 0.015 µM. In other embodiments, compound of formula (I) may be administered at about 0.02 µM. In other embodiments, compound of formula (I) may be administered at about 0.025 µM. In other embodiments, compound of formula (I) may be administered at about 0.027 µM. In other embodiments, compound of formula (I) may be administered at about 0.03 µM. In other embodiments, compound of formula (I) may be administered at about 0.05 µM. In other embodiments, compound of formula (I) may be administered at about 0.07 µM. In other embodiments, compound of formula (I) may be administered at about 0.1 µM. In other embodiments, compound of formula (I) may be administered at about 0.15 µM. In other embodiments, compound of formula (I) may be administered at about 0.2 µM. In other embodiments, compound of formula (I) may be administered at about 0.25 µM. In other embodiments, compound of formula (I) may be administered at about 0.29 µM. In other embodiments, compound of formula (I) may be administered at about 0.3 µM. In other embodiments, compound of formula (I) may be administered at about 0.5 µM. In other embodiments, compound of formula (I) may be administered at about 0.7 µM. In other embodiments, compound of formula (I) may be administered at about 0.9 µM. In other embodiments, compound of formula (I) may be administered at about 1.1 µM. In other embodiments, compound of formula (I) may be administered at about 1.5 µM. In other embodiments, compound of formula (I) may be administered at about 2 µM. In other embodiments, compound of formula (I) may be administered at about 4 µM. In other embodiments, compound of formula (I) may be administered at about 6 µM. In other embodiments, compound of formula (I) may be administered at about 8 µM. In other embodiments, compound of formula (I) may be administered at about 10 µM. In other embodiments, compound of formula (I) may be administered at about 12 µM. In other embodiments, compound of formula (I) may be administered at about 14 µM. In other embodiments, compound of formula (I) may be administered at about 24 µM. In other embodiments, compound of formula (I) may be administered at about 36 µM. In other embodiments, compound of formula (I) may be administered at about 48 µM. In other embodiments, compound of formula (I) may be administered at about 60 µM.

### Combination partner (b): PARP inhibitor

As used herein, "PARP inhibitor" is an inhibitor that acts on the enzyme poly(ADP-ribose) polymerase (PARP). Examples of known PARP inhibitors are Niraparib (MK-4827), Olaparib and Rucaparib.

DNA is damaged thousands of times during each cell cycle, and that damage must be repaired, including in cancer cells. Otherwise, the cells may die due to this damage. For example, BRCA1, BRCA2 and PALB2 are proteins that are important for the repair of double-strand DNA breaks by the error-free homologous recombinational repair pathway. When the gene for any one of these proteins is mutated, the change can lead to errors in DNA repair that can eventually cause breast cancer. When subjected to enough damage at one time, the altered gene can cause the death of the cells.

During DNA damage, PARP detects the single-stranded DNA breaks and activates the repair mechanism. Hence, when a PARP inhibitor is introduced, the repair is stalled and eventually causes further breaks and genomic instability leading to cell death.

It is believed that inhibition of PARP can cause multiple double strand breaks to form in this way, and in tumours with BRCA1, BRCA2 or PALB2 mutations, these double strand breaks cannot be efficiently repaired, leading to the death of the cells. In this sense, PARP inhibitors are thought to work by blocking the DNA repair function of members of the PARP family of proteins, leading ultimately to cancer cell death. Further, some cancer cells that lack the tumor suppressor PTEN may be sensitive to PARP inhibitors because of downregulation of Rad51, a critical homologous recombination component. PARP inhibitors may be effective against many PTEN-defective tumours (e.g. some aggressive prostate cancers). Cancer cells that are low in oxygen (e.g. in fast growing tumors) are sensitive to PARP inhibitors.

It is also believed that PARP inhibitors may be categorized according to their potency to trap PARP, in addition to their ability to inhibit DNA repair. These trapped PARP complexes are believed to be toxic to the cells. By binding the PARP inhibitor to PARP, the PARP proteins are localised at sites of DNA damage. The trapped PARP protein-DNA complexes are highly toxic to cells because they block DNA replication. The PARP family of proteins in humans includes PARP1 and PARP2, which are DNA binding and repair proteins. When activated by DNA damage, these proteins recruit other proteins that do the actual work of repairing DNA. Under normal conditions, PARP1 and PARP2 are released from DNA once the repair process is underway. However, when they are bound to PARP inhibitors, PARP1 and PARP2 become trapped on DNA. Trapped PARP-DNA complexes are more toxic to cells than the unrepaired single-strand DNA breaks that accumulate in the absence of PARP activity, suggesting that PARP inhibitors may also act as PARP poisons.

According to the invention, the PARP inhibitor is Olapraib. Olaparib (AZD-2281; trade name Lynparza) is shown below as the compound of formula (II) and in the context of the present invention encompasses its pharmaceutically acceptable salts, or solvates thereof

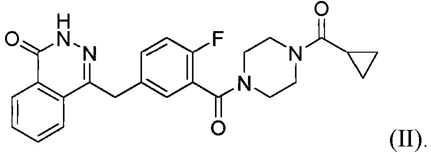

In some embodiments, compound of formula (II) may be administered at about 0.25 times of its IC₅₀ value. In other embodiments, compound of formula (II) may be administered at about 0.5 times of its IC₅₀ value. In other embodiments, compound of formula (II) may be administered at about 0.75 times of its IC₅₀ value. In other embodiments, compound of formula (II) may be administered at about its IC₅₀ value. In other embodiments, compound of formula (II) may be administered at about 1.25 times of its IC₅₀ value. In other embodiments, compound of formula (II) may be administered at about 1.5 times of its IC₅₀ value. In other embodiments, compound of formula (II) may be administered at about 1.75 times of its IC₅₀ value. In other embodiments, compound of formula (II) may be administered at about 2 times of its IC₅₀ value. In other embodiments, compound of formula (II) may be administered at about 3 times of its IC₅₀ value. In other embodiments, compound of formula (II) may be administered at about 4 times of its IC₅₀ value. In other embodiments, compound of formula (II) may be administered at about 5 times of its IC₅₀ value.

In some embodiments, compound of formula (II) may be administered at about 0.25 times of its EC₅₀ value. In other embodiments, compound of formula (II) may be administered at about 0.5 times of its EC₅₀ value. In other embodiments, compound of formula (II) may be administered at about 0.75 times of its EC₅₀ value. In other embodiments, compound of formula (II) may be administered at about its EC₅₀ value. In other embodiments, compound of formula (II) may be administered at about 1.25 times of its EC₅₀ value. In other embodiments, compound of formula (II) may be administered at about 1.5 times of its EC₅₀ value. In other embodiments, compound of formula (II) may be administered at about 1.75 times of its EC₅₀ value. In other embodiments, compound of formula (II) may be administered at about 2 times of its EC₅₀ value. In other embodiments, compound of formula (II) may be administered at about 3 times of its EC₅₀ value. In other embodiments, compound of formula (II) may be administered at about 4 times of its EC₅₀ value. In other embodiments, compound of formula (II) may be administered at about 5 times of its EC₅₀ value.

In some embodiments, compound of formula (II) may be administered at about 0.1 µM. In other embodiments, compound of formula (II) may be administered at about 0.25 µM. In other embodiments, compound of formula (II) may be administered at about 0.5 µM. In other embodiments, compound of formula (II) may be administered at about 0.75 µM. In other embodiments, compound of formula (II) may be administered at about 1 µM. In other embodiments, compound of formula (II) may be administered at about 2 µM. In other embodiments, compound of formula (II) may be administered at about 3 µM. In other embodiments, compound of formula (II) may be administered at about 4 µM. In other embodiments, compound of formula (II) may be administered at about 5 µM. In other embodiments, compound of formula (II) may be administered at about 6 µM. In other embodiments, compound of formula (II) may be administered at about 8 µM. In other embodiments, compound of formula (II) may be administered at about 9 µM. In other embodiments, compound of formula (II) may be administered at about 10 µM. In other embodiments, compound of formula (II) may be administered at about 12 µM. In other embodiments, compound of formula (II) may be administered at about 14 µM. In other embodiments, compound of formula (II) may be administered at about 16 µM. In other embodiments, compound of formula (II) may be administered at about 18 µM. In other embodiments, compound of formula (II) may be administered at about 20 µM. In other embodiments, compound of formula (II) may be administered at about 30 µM. In other embodiments, compound of formula (II) may be administered at about 40 µM. In other embodiments, compound of formula (II) may be administered at about 50 µM. In other embodiments, compound of formula (II) may be administered at about 75 µM. In other embodiments, compound of formula (II) may be administered at about 100 µM. In other embodiments, compound of formula (II) may be administered at about 125 µM. In other embodiments, compound of formula (II) may be administered at about 150 µM. In other embodiments, compound of formula (II) may be administered at about 175 µM.

### The combination of (a) and (b)

It will be appreciated that compounds of formula I and/or II can be administered to a subject as a pharmaceutically acceptable salt thereof. Suitable pharmaceutically acceptable salts include, but are not limited to salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, ammonium and alkylammonium. In particular, the present invention includes within its scope cationic salts eg sodium or potassium salts, or alkyl esters (eg methyl, ethyl) of the phosphate group.

The compounds of formula I and/or II (or a salt thereof) may be in crystalline form either as the free compound or as a solvate (e.g. hydrate) and it is intended that both forms are within the scope of the present invention. Methods of solvation are generally known within the art.

Without wishing to be bound to any particular theory, it is believed that partners (a) and (b) work in combination to better effect cancer cell death or senescence. As mentioned, the inhibition of Wnt signalling using a compound of Formula (I) downregulates expression of DNA repair genes. PARP inhibition selectively induces cell death in cancer cells by blocking DNA repair mechanisms. It is postulated that the combination of a PARP inhibitor forming a complex with the DNA strand and a Wnt modulator acting on PORCN reduces inter- and intra-cell signalling to selectively induce cell death in cancer cells, thereby yielding a beneficial additive or synergistic effect. Accordingly, the non-selective and non-specific effect of each individual inhibitor is reduced. Further, PARP-1 overexpression appears to be positively correlated with the overexpression of β-catenin. PARP-1 is also believed to be a co-enhancer of β-catenin-evoked gene transcription. It is postulated that PARP-1 can influence the downstream activities of the WNT pathway. Accordingly, by targeting the WNT pathway with two different inhibitors (PARP and PORCN), it is envisioned that at least a combinatory (additive or synergistic) effect can be achieved.

In some embodiments, partner (a) is administered before partner (b). In other embodiments, partner (a) is administered concurrently with partner (b). In other embodiments, partner (a) is administered at least 2 h before partner (b). In other embodiments, partner (a) is administered at least 4 h, 6 h, 8 h, 10 h, 12 h, 24 h, 36 h, 48 h, or 56 h before partner (b).

Advantageously, the combination treatment using a compound of Formula (I) with olaparib would reduce the dose of each inhibitor to be administered, thereby reducing the potential non-selective and non-specific effects of each individual inhibitor.

It has been found that the combination of (a) compound of formula (I) and (b) compound of formula (II) yields a synergistic effect in the treatment of pancreatic cancer, ovarian cancer and cholangio-carcinomas. In these cell lines, the combination index values obtained using the Compusyn software were found to be less than 0.9, indicating that the combination is synergistic (wherein a value of <0.9 is indicative of synergism). In certain embodiments, the combination was found to be effective in inhibiting pancreatic cancer growth.

To assess the IC₅₀ and EC₅₀ of the cell lines, the cell lines are treated with individual drugs at concentrations up to 10-100uM. The IC₅₀ and EC₅₀ values for each combination partner (a) and (b) with the specific cell lines are determined from the response plot. A dose-response assay for each combination partner and the combination at different concentrations based on the IC₅₀ values of each combination partner was performed. Computed EC₅₀-EC₉₅ values were then generated by a software (as disclosed herein) based on the drug concentration and the respective colony growth numbers.

In an embodiment, a ratio of a EC₅₀ value of compound of formula (I) to a EC₅₀ value of compound of formula (II), wherein the ratio is from about 5:1 to about 1:5 is disclosed. In another embodiment, the ratio is from about 4:1 to about 1:4. In another embodiment, the ratio is from about 3:1 to about 1:3. In another embodiment, the ratio is from about 2:1 to about 1:2. In certain embodiments, the ratio is about 1:1.

In an embodiment, the ratio of a EC₅₀ value of compound of formula (I) to a EC₅₀ value of compound of formula (II) is about 5:1. In another embodiment, the ratio is about 4:1. In another embodiment, the ratio is about 3:1. In another embodiment, the ratio is about 2:1. In another embodiment, the ratio is about 1:1. In another embodiment, the ratio is about 1:2. In another embodiment, the ratio is about 1:3. In another embodiment, the ratio is about 1:4. In another embodiment, the ratio is about 1:5.

In some embodiments, when the ratio of a EC₅₀ value of compound of formula (I) to a EC₅₀ value of compound of formula (II) is 1:1, both compounds are each administered at a different IC₅₀ value. For example, compound of formula (I) can be administered at about 0.5 times its IC₅₀ value while compound of formula (II) can be administered at about 0.25 times its IC₅₀ value.

In some embodiments, when the ratio of a EC₅₀ value of compound of formula (I) to a EC₅₀ value of compound of formula (II) is 1:1, both compounds are each administered at about 0.25 times their respective IC₅₀ value. In other embodiments, both compounds are each administered at about 0.5 times their respective IC₅₀ value. In other embodiments, both compounds are each administered at about 0.75 times their respective IC₅₀ value. In other embodiments, both compounds are each administered at about 1.0 times their respective IC₅₀ value. In other embodiments, both compounds each administered at about 1.25 times their respective IC₅₀ value. In other embodiments, both compounds are each administered at about 1.5 times their respective IC₅₀ value. In other embodiments, both compounds are each administered at about 1.75 times their respective IC₅₀ value. In other embodiments, both compounds are each administered at about 2.0 times their respective IC₅₀ value. In other embodiments, both compounds are each administered at about 3.0 times their respective IC₅₀ value. In other embodiments, both compounds are each administered at about 4.0 times their respective IC₅₀ value. In other embodiments, both compounds are each administered at about 5.0 times their respective IC₅₀ value.

In some embodiments, when the ratio of a EC₅₀ value of compound of formula (I) to a EC₅₀ value of compound of formula (II) is 1:1, both compounds are each administered at about 0.25 times their respective EC₅₀ value. In other embodiments, both compounds are each administered at about 0.5 times their respective EC₅₀ value. In other embodiments, both compounds are each administered at about 0.75 times their respective EC₅₀ value. In other embodiments, both compounds are each administered at about 1.0 times their respective EC₅₀ value. In other embodiments, both compounds each administered at about 1.25 times their respective EC₅₀ value. In other embodiments, both compounds are each administered at about 1.5 times their respective EC₅₀ value. In other embodiments, both compounds are each administered at about 1.75 times their respective EC₅₀ value. In other embodiments, both compounds are each administered at about 2.0 times their respective EC₅₀ value. In other embodiments, both compounds are each administered at about 3.0 times their respective EC₅₀ value. In other embodiments, both compounds are each administered at about 4.0 times their respective EC₅₀ value. In other embodiments, both compounds are each administered at about 5.0 times their respective EC₅₀ value. In another embodiment, both compounds are each administered at a different factor of their respective EC₅₀ value.

An "effective amount" is intended to mean that the amount of each combination partner, when administered to a mammal (in particular a human) in need of such treatment, is sufficient to effect treatment for a particular cancer. Thus, for example, a therapeutically effective amount of a compound of combination partner (a) (or a pharmaceutically acceptable salt, solvate, or prodrug thereof) may be a quantity sufficient to synergise or potentiate the activity of the PARP inhibitor (or *vice versa*) such that a targeted disease is reduced or alleviated. It may also be an amount that provides an additive effect.

This may include at least partially attaining the desired effect, or delaying the onset of, or inhibiting the progression of, or halting or reversing altogether the onset or progression of the particular disease (e.g., tumour) being treated.

Clinical studies such as open-label, dose escalation studies in patients with cancers may include studies to prove the synergism of the active ingredients of the combination. The beneficial and/or synergistic effects can be determined directly through the results of these studies which are known as such to a person skilled in the art. These studies are also able to compare the effects of a monotherapy using the active ingredients and a combination of the invention. Preferably, the dose of combination partner (a) may be escalated until the Maximum Tolerated Dosage (MTD) is reached, and agent (b) is administered as a fixed dose. Alternatively, combination partner (a) is administered in a fixed dose and the dose of agent (b) is escalated. Each patient may receive doses of agent (a) either daily or intermittently. The efficacy of the treatment can be determined in such studies, e.g., after 6, 12, 18 or 24 weeks by evaluation of symptom scores every 9 weeks.

The administration of the pharmaceutical combination of the present invention may result not only in a beneficial effect, e.g., an additive or synergistic therapeutic effect, for instance, with regard to alleviating, delaying progression of or inhibiting the symptoms, but also in further surprising beneficial effects. Such other effects may include fewer side effects, an improved quality of life or a decreased morbidity, compared with a monotherapy applying only one of the pharmaceutically active ingredients used in the combination of the present invention.

A further benefit of the invention is that lower doses of the active ingredients of the combination can be used. The dosages need not only be smaller but may also be applied less frequently, which may diminish the incidence or severity of side effects.

The term "administration" relates to the co-administration of the combination partners to a single patient, and is intended to include treatment regimens in which the agents are not necessarily administered by the same route of administration or at the same time. Accordingly, combination partners (a) and (b) may be administered together, one after the other or separately in one combined unit dosage form or in two separate unit dosage forms. The unit dosage form may also be a fixed combination such as a pharmaceutical composition which comprises both partner (a) (or a salt, solvate or prodrug thereof) and partner (b).

In particular, a therapeutically effective amount of each of the combination partners of the combination of the invention may be administered simultaneously or sequentially in any order, and the components may be administered separately or as a fixed combination.

For example, the method of preventing or treating cancers may comprise: (i) administration of partner (a) in free or pharmaceutically acceptable salt form; and (ii) administration of partner (b) in free or pharmaceutically acceptable salt form, simultaneously or sequentially in any order, in jointly therapeutically effective amounts, preferably in synergistically effective amounts, e.g., in daily or intermittent dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that converts *in vivo* to the combination partner as such. The present invention is therefore to be understood as embracing all such regimens of simultaneous or alternating treatment and the term "administering" is to be interpreted accordingly.

As such it will be appreciated that the combination partners may be presented as a "kit of parts" for use in the treatment of a cancer (e.g., tumour therapy). The kit may comprise a package where the combination partners are supplied separately for co-administration with instructions for use in the particular therapy.

The effective dosage of each of the combination partners employed in the combination of the invention may vary depending on the particular compound or pharmaceutical composition employed, the mode of administration, the condition being treated and the severity of the condition being treated. Thus, the dosage regimen of the combination of the invention is selected in accordance with a variety of factors including the route of administration and the renal and hepatic function of the patient. A physician of ordinary skill can readily determine and prescribe the effective amount of the single active ingredients required to alleviate, counter or arrest the progress of the condition.

Daily dosages for combination partners (a) and (b) will, of course, vary depending on a variety of factors, e.g., the compound chosen, the particular condition to be treated and the desired effect. In general, however, satisfactory results are achieved on administration of partner (a) at daily dosage rates of about 0.05 to 20 mg/kg per day, particularly 1 to 20 mg/kg per day, e.g. 0.4 to 16 mg/kg per day, as a single dose or in divided doses. Combination partner (a) and partner (b) may be administered by any conventional route, in particular enterally, e.g., orally, e.g., in the form of tablets, capsules, drink solutions or parenterally, e.g., in the form of injectable solutions or suspensions. Suitable unit dosage forms for oral administration comprise from about 0.02 to 50 mg of each active ingredient (combination partner (a) or (b)), or about 0.1 to 30 mg, or about 2 to 25 mg, or about 4 to 20 mg, together with one or more pharmaceutically acceptable diluents or carriers therefore.

Combination partner (b) may be administered to a human in a daily dosage range of 0.5 to 1000 mg. Suitable unit dosage forms for oral administration comprise from about 0.1 to 500 mg active ingredient, together with one or more pharmaceutically acceptable diluents or carriers therefore. Methods and administration regimes for delivery of olaparib would be known to the skilled clinican.

For instance, an administration regime may include adding the compound of formula (I) at an assigned dose level by I.V. on days 1 and 8 (of a 21 day cycle) where the PARP inhibitor (e.g., compound of formula II) is given as an oral daily dose (e.g., 10 mg/day). In this embodiment the compound of formula (I) may be dosed at a level of between 2 to 24 mg/kg.

In an embodiment, when compound of formula (II) is adminisitered at about 100 mg to about 400 mg per dose, compound of formula (I) is administered at about 50 mg to about 2000 mg per dose. In another embodiment, compound of formula (I) is administered at about 60 mg to about 1800 mg per dose. In another embodiment, compound of formula (I) is administered at about 70 mg to about 1600 mg per dose. In another embodiment, compound of formula (I) is administered at about 80 mg to about 1400 mg per dose. In another embodiment, compound of formula (I) is administered at about 90 mg to about 1200 mg per dose. In another embodiment, compound of formula (I) is administered at about 100 mg to about 1000 mg per dose. In another embodiment, compound of formula (I) is administered at about 100 mg to about 800 mg per dose. In another embodiment, compound of formula (I) is administered at about 100 mg to about 600 mg per dose. In another embodiment, compound of formula (I) is administered at about 100 mg to about 500 mg per dose. In another embodiment, compound of formula (I) is administered at about 100 mg to about 400 mg per dose. In another embodiment, compound of formula (I) is administered at about 100 mg to about 300 mg per dose.

The administration of a pharmaceutical combination of the invention results not only in a beneficial effect, e.g., an additive or synergistic therapeutic effect, e.g., with regard to inhibiting the growth of tumors, but also in further surprising beneficial effects, e.g., fewer side effects, an improved quality of life or a decreased morbidity, compared to a monotherapy applying only one of the pharmaceutically active ingredients used in the combination of the invention.

A further benefit is that lower doses of the active ingredients of the combination of the invention can be used, e.g., the dosages often may not only be smaller, but also applied less frequently, or can be used in order to diminish the incidence of side effects. This is in accordance with the desires and requirements of the patients to be treated.

Combinations of partners (a) and (b) may be combined, independently or together, with one or more pharmaceutically acceptable carriers and, optionally, one or more other conventional pharmaceutical adjuvants and administered enterally, e.g., orally, in the form of tablets, capsules, caplets, etc. or parenterally, e.g., intraperitoneally or intravenously, in the form of sterile injectable solutions or suspensions. The enteral and parenteral compositions may be prepared by conventional means.

The pharmaceutical compositions for separate administration of combination partner (a) and partner (b) or for the administration in a fixed combination (i.e., a composition), according to the invention may be prepared in a manner known in the art and are those suitable for enteral, such as oral or rectal, and parenteral administration to mammals (warm-blooded animals), particularly humans, comprising a therapeutically effective amount of at least one pharmacologically active combination partner alone, e.g., as indicated above, or in combination with one or more pharmaceutically acceptable carriers or diluents, especially suitable for enteral or parenteral application.

Suitable pharmaceutical compositions contain, e.g., from about 0.1% to about 99.9%, preferably from about 1 % to about 60%, of the active ingredient(s).

The composition may contain any suitable carriers, diluents or excipients. These include all conventional solvents, dispersion media, fillers, solid carriers, coatings, antifungal and antibacterial agents, dermal penetration agents, surfactants, isotonic and absorption agents and the like. It will be understood that the compositions of the invention may also include other supplementary physiologically active agents.

The carrier must be pharmaceutically "acceptable" in the sense of being compatible with the other ingredients of the composition and not injurious to the subject. Compositions include those suitable for oral, rectal, nasal, topical (including buccal and sublingual), vaginal or parenteral (including subcutaneous, intramuscular, intravenous and intradermal) administration. The compositions may conveniently be presented in unit dosage form and may be prepared by any methods well known in the art of pharmacy. Such methods include the step of bringing into association the active ingredient with the carrier which constitutes one or more accessory ingredients. In general, the compositions are prepared by uniformly and intimately bringing into association the active ingredient with liquid carriers or finely divided solid carriers or both, and then if necessary shaping the product.

Compositions of the present invention suitable for oral administration may be presented as discrete units such as capsules, sachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g inert diluent), preservative disintegrant (e.g. sodium starch glycolate, cross-linked polyvinyl pyrrolidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Compositions suitable for topical administration in the mouth include lozenges comprising the active ingredient in a flavoured base, usually sucrose and acacia or tragacanth gum; pastilles comprising the active ingredient in an inert basis such as gelatine and glycerin, or sucrose and acacia gum; and mouthwashes comprising the active ingredient in a suitable liquid carrier.

Compositions suitable for topical administration to the skin may comprise the compounds dissolved or suspended in any suitable carrier or base and may be in the form of lotions, gel, creams, pastes, ointments and the like. Suitable carriers include mineral oil, propylene glycol, polyoxyethylene, polyoxypropylene, emulsifying wax, sorbitan monostearate, polysorbate 60, cetyl esters wax, cetearyl alcohol, 2-octyldodecanol, benzyl alcohol and water. Transdermal patches may also be used to administer the compounds of the invention.

Compositions for rectal administration may be presented as a suppository with a suitable base comprising, for example, cocoa butter, glycerin, gelatine or polyethylene glycol.

Compositions suitable for vaginal administration may be presented as pessaries, tampons, creams, gels, pastes, foams or spray formulations containing, in addition to the active ingredient, such carriers as are known in the art to be appropriate.

Compositions suitable for parenteral administration include aqueous and non-aqueous isotonic sterile injection solutions which may contain anti-oxidants, buffers, bactericides and solutes which render the composition isotonic with the blood of the intended recipient; and aqueous and non-aqueous sterile suspensions which may include suspending agents and thickening agents. The compositions may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage compositions are those containing a daily dose or unit, daily sub-dose, as herein above described, or an appropriate fraction thereof, of the active ingredient.

It should be understood that in addition to the active ingredients particularly mentioned above, the compositions of this invention may include other agents conventional in the art having regard to the type of composition in question, for example, those suitable for oral administration may include such further agents as binders, sweeteners, thickeners, flavouring agents, disintegrating agents, coating agents, preservatives, lubricants and/or time delay agents. Suitable sweeteners include sucrose, lactose, glucose, aspartame or saccharine. Suitable disintegrating agents include cornstarch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Those skilled in the art will appreciate that the invention described herein is susceptible to variations and modifications other than those specifically described. It is to be understood that the invention includes all such variations and modifications which fall within the spirit and scope. The invention also includes all of the steps, features, compositions and compounds referred to or indicated in this specification, individually or collectively, and any and all combinations of any two or more of said steps or features.

Certain embodiments of the invention will now be described with reference to the following examples which are intended for the purpose of illustration only.

### Examples

### Synthesis of compound of formula (I)

### Synthesis of 6-phenylpyridazin-3-amine:

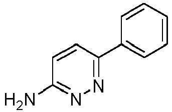

A stirred solution of the arylhalide (1 equiv.), boronic acid (1.5 equiv.) and sodium carbonate (2 equiv.) in toluene (0.08 M) and water (0.32 M) was degassed for 15 min with argon. Tetrakis(triphenylphosphine)palladium(0) (0.05 equiv.) was added to reaction mixture and the reaction mixture was heated to reflux for 16 h. After completion of starting material, the reaction mixture was concentrated and water was added to reaction mixture and extracted with ethyl acetate. The combined organic layers were washed with brine, dried with Na₂SO₄ and concentrated under vacuum. The crude compound was purified by column chromatography to afford the purified product.

1H NMR (400 MHz, DMSO-d6) δ (ppm): 7.96-7.94 (d, J=8 Hz, 2H), 7.82-7.80 (d, J=9.2 Hz, 1H), 7.48-7.35 (m, 3H), 6.86-6.84 (m, 1H), 6.64(br s, 2H). LC-MS: m/z 172.0 (M+H) with a purity of 82%.

### Synthesis of 2-(1,3-dimethyl-2,6-dioxo-1,2,3,6-tetrahydro-7H-purin-7-yl)-N-(6-phenylpyridazin-3-yl)acetamide (alternatively named 2-(1,3-dimethyl-2,6-dioxo-2,3-dihydro-1H-purin-7(6H)-yl)-N-(6-phenyl pyridazin-3-yl)acetamide) (Ex 1):

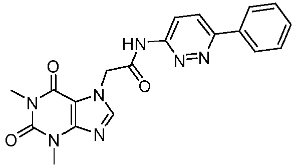

To a stirred solution of commercially available 2-(1,3-dimethyl-2,6-dioxo-2,3-dihydro-1H-purin-7(6H)-yl)acetic acid, 1 (1 equiv.) in dichloromethane (0.01 M) was added HATU (1.3 equiv.), triethylamine (1.5 equiv.) and the respective amine (1 equiv.). The reaction mixture was allowed to stir at room temperature. Upon completion of the reaction, water was added to the reaction mixture and the mixture was extracted with dichloromethane. The organic layer was washed with brine solution, dried over anhydrous Na₂SO₄, and concentrated under vacuum to afford the crude product. The crude product is further purified by column chromatography.

1H NMR (400 MHz, DMSO-d6) δ (ppm): 11.77 (s, 1H), 8.32-8.25 (m, 2H), 8.12-8.10 (m, 3H), 7.57-7.49 (m, 3H), 5.37 (s, 2H), 3.46 (s, 3H), 3.19 (s, 3H). LC-MS: m/z 390.1 (M+H) with a purity of 99%.

### Compound of formula (II)

Compound of formula (II) may be bought from Selleckchem (Olaparib; AZD2281, Ku-0059436; Catalog No.S1060).

### Biological Studies for the Combinations

### Materials

1. 3 % Agar (Sigma; A1296- 500G): dissolve 3 grams of agar powder in 100 ml of milliQ water. Sterilize the agar by autoclaving and store it at room temperature.
2. 5 mg/ml 3-(4,5-Dimethyl-2-thiazolyl)-2,5-diphenyl-2H-tetrazolium bromide (MTT) staining solution: dissolve 250 mg MTT powder (Sigma cat# M5655) in 50 ml of PBS, make aliquots and store them at -20°C.
3. Compound of formula (I) and Olaparib (AZD2281, Selleckchem: Catalog No.S1060): dissolve compounds in DMSO and store them at -20°C as 10 mM stocks.
4. 48-well cell culture plates (Greiner; 677102): only use 36 wells per plate. Fill the first and last columns of wells with 500 µl of sterile water or phosphate buffered saline.
5. Set the water bath at 37°C. Warm all media in water bath.
6. Cell lines used and number of cells seeded per well

| Cell Lines | Media | Cells seeded per well |
|---|---|---|
| EGI-1 | MEM + 10% FBS | 10,000 |
| HPAF-II | MEM + 10% FBS | 7000 |
| MCAS | MEM + 20% FBS | 6000 |
| CFPAC-1 | IMDM + 10% FBS | 5000 |
| TFK-1 | RPMI + 10% FBS | 5000 |
| PA-TU-8988T | MEM + 5% FBS + 5% Horse Serum | 5000 |

### Procedure

### (1) Base Layer - 0.6% Agar per 300µL per well.

For 2 plates: 5.4 ml 3% agar + 21.6 ml media (Total volume 27 ml)

Microwave the agar for 1-2 min so that it is completely melted. Add 5.4 ml of the melted agar into 21.6 ml of pre-warmed media (All media and agar preparation is made in excess for 2.5 plates). Aliquot 300 µl of the suspension into each well, avoiding the first and last columns of the plates. Fill the first and last columns of wells with 500 µl of sterile water or phosphate buffered saline. Incubate the plates in 37°C incubator for 45min - 1hr to allow agar to solidify.

### (2) Middle Layer - 0.3% Agar plus corresponding number of cells per well

For 2 plates: 2.43ml of 3% agar + 20.07 ml media with cells (Total vol. 22.5ml)

Trypsinize the adherent cells and suspend them in 5 ml of media. Determine the number of cells in the suspension and calculate the volume of cell suspension required for seeding. Mix the agar and media together first, excluding the volume of cell suspension. Add the cell suspension last into the mixture. Mix well and aliquot 250 µl into each well containing the base layer. Incubate the plates in a 37°C incubator for 1- 1.5 hrs to allow the agar to solidify.

### (3) Top Layer - 6 drug dose points: 0x, 0.25x, 0.5x, 1x, 2x and 4x IC50.

Set up plate as shown below.

Prepare the required concentration of compounds based on the IC50 values of each drug. (Pre-determine the IC50 values for each drug with the corresponding cell-line using a dose-response assay)
Row A& B: Aliquot 250 µl of diluted Drug A per well
Row C&D: Aliquot 250 µl of diluted Drug B per well
Row E& F: Aliquot 250 µl of diluted Drug A and B mixed at 1:1 ratio for each well

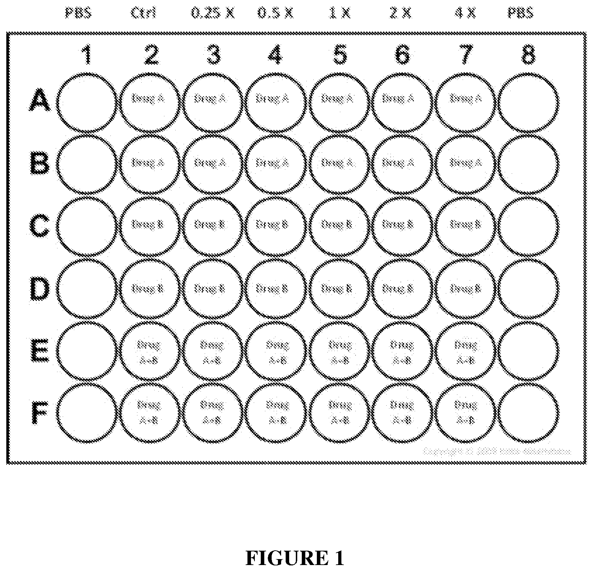

Figure 1 illustrates an example of a dose-response assay plate for testing compounds at different concentrations based on the IC₅₀ values of each compound.

Assay results analysis method
1. Score the colonies after 2 to 3 weeks depending on the colony forming status for different cell line.
2. Before scoring the colonies, add 35 µl MTT (5 mg/ml) to the wells with cells and incubate at 37°C for 2-3 hrs depending on the intensity of colour development.
3. Once the colonies are stained, count the total number of colonies formed using the Oxford Optronix GelCount^{®} instrument.
4. Average the total number of colonies obtained for each drug dose point and represent it as a fraction over the control. This represents the fraction of colony growth. Hence, fraction of inhibition = 1 - fraction of colony growth.
5. Incorporate the fraction of inhibition and the corresponding dose values into the Chou Talalay Software available online (http://www.combosyn.com/) to obtain the combination index values.
6. The combination index values will give an indication as to whether the combined drugs are synergistic, antagonistic or additive in the treated cells, as shown in Table 2 below.

**Table 2: Combination Index and type of combination effect**

| **Range of Combination Index** | **Description** |
|---|---|
| **<0.1** | **Very strong synergism** |
| **0.1-0.3** | **Strong synergism** |
| **0.3-0.7** | **Synergism** |
| **0.7-0.85** | **Moderate synergism** |
| **0.85-0.90** | **Slight synergism** |
| **0.90-1.10** | **Nearly additive** |
| **1.10-1.20** | **Slight antagonism** |
| **1.20-1.45** | **Moderate antagonism** |
| **1.45-3.3** | **Antagonism** |
| **3.3-10** | **Strong antagonism** |
| **>10** | **Very strong antagonism** |

### Mouse Xenograft model

Mouse xenograft model were established by subcutaneous injection of HPAF-II cells in NSG mice. 5x10⁶ HPAF-II cells resuspended in 50% matrigel were used for injection. Mice were treated with compound of Formula (I) and/or Olaparib after establishment of tumors. Compound of Formula (I) was formulated in 50% PEG-400 (vol/vol) in water and administerd by oral gavage at a dosing volume of 10 µL/g body weight. The tumor dimensions were measured with a caliper routinely, and the tumor volumes were calculated as 0.5 x length x width². All mice were sacrified 8 hr after the last dosing.

### RNA Isolation and qRT-PCR

Tumors were homogenised in RLT buffer using a polytron homogenizer. Total RNA was isolated using RNAeasy kit (Qiagen, Germany) according to manufacturer's protocol. For qPT-PCR, RNA was reverse transcribed with iScript reverse transcriptase (BioRAD, Hercules, CA, USA). Real time quantitative PCR (qPCR) was performed using SsoFast EvaGreen assay Supermix from BioRad (Hercules, CA, USA). *EPN1* and *ACTB* were used as housekeeping genes. The primers used are listed in the Table below.

| | |
|---|---|
| BRCA1-F | 5'-CAACATGCCCACAGATCAAC-3' |
| BRCA1-R | 5'-ATGGAAGCCATTGTCCTCTG-3' |
| BRCA2-F | 5'-CAGAAGCCCTTTGAGAGTGG-3' |
| BRCA2-R | 5'-TCCATCTGGGCTCCATTTAG-3" |
| FANCD2-F | 5'-CCCTGAGCTGCTTTTCTTGC-3' |
| FANCD2-R | 5'-CGGCTTCCTTTGTTCTTGAG-3' |
| FANCG-F | 5'-CTGTTCTTCCCTTGGAGCTG-3' |
| FANCG-R | 5'-TCTCTAGGCTCCGCTGGATA-3' |
| RAD51-F | 5'-TTTGGAGAATTCCGAACTGG-3' |
| RAD51-R | 5'-TACATGGCCTTTCCTTCACC-3' |
| XRCC3-F | 5'-GTGCATCAACCAGGTGACAG-3' |
| XRCC3-R | 5'-TTAGCCCAGGTTATGCCAAG-3' |

### Results

### Example 1) EGI-1 cells (Test 1)

| | | | | | | | |
|---|---|---|---|---|---|---|---|
| **Olaparib** | **Dose** | **0** | **2.16362** | **4.32725** | **8.6545** | **17.309** | **34.618** |
| **IC₅₀ = 8.65µM** | **Colony count** | 2033 | 1658 | 1362 | 935 | 464 | 209 |
| | | 1955 | 1605 | 1300 | 908 | 489 | 197 |
| **Ex 1** | **Dose** | **0** | **0.00687** | **0.01375** | **0.0275** | **0.055** | **0.11** |
| **IC₅₀ = 0.0275 µM** | **Colony count** | 2054 | 1742 | 1351 | 955 | 608 | 515 |
| | | 2020 | 1591 | 1351 | 912 | 646 | 465 |
| **Olaparib+Ex 1** | **Colony count** | 1879 | 1085 | 542 | 170 | 42 | 26 |
| | | 1684 | 907 | 469 | 152 | 52 | 23 |

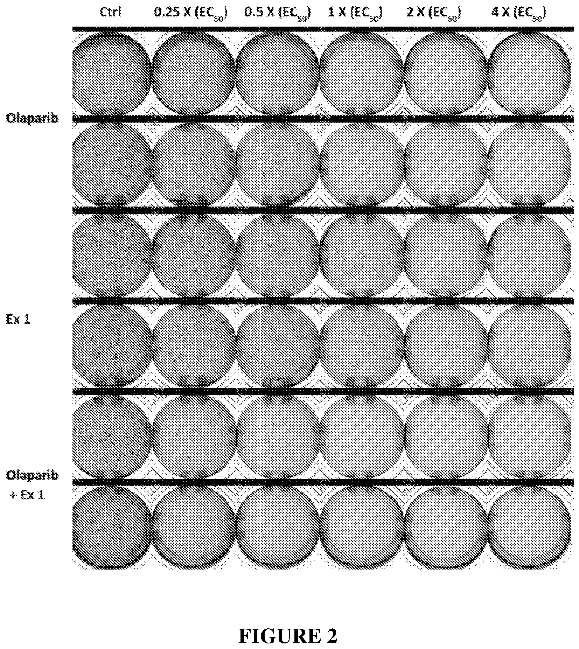

Figure 2 illustrates a dose-response assay plate containing EGI-1 cells (Test 1) after testing compounds at different concentrations based on the IC₅₀ values of each compound. EGI-1 is a cell line established from cholangiocarcinoma (bile duct adenocarcinoma).

### EGI-1 Cells (Test 2)

| | | | | | | | |
|---|---|---|---|---|---|---|---|
| **Olaparib** | **Dose** | **0** | **2.16362** | **4.32725** | **8.6545** | **17.309** | **34.618** |
| **IC₅₀ = 8.65 µM** | **Colony count** | 1995 | 1591 | 1403 | 856 | 446 | 144 |
| | | 2012 | 1717 | 1442 | 1052 | 482 | 198 |
| **Ex 1** | **Dose** | **0** | **0.00687** | **0.01375** | **0.0275** | **0.055** | **0.11** |
| **IC₅₀ = 0.0275 µM** | **Colony count** | 1785 1740 | 1488 1451 | 1242 1139 | 816 771 | 568 476 | 434 412 |
| **Olaparib+Ex 1** | **Colony count** | 2095 2009 | 1322 1054 | 720 581 | 258 160 | 58 59 | 24 34 |

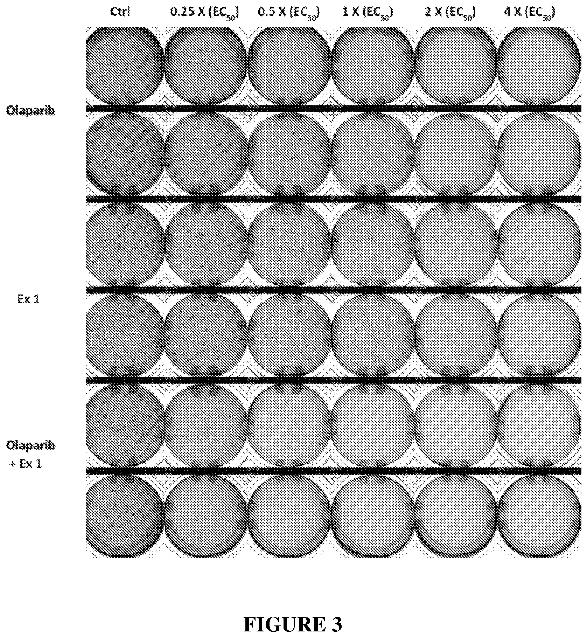

Figure 3 illustrates a dose-response assay plate containing EGI-1 cells (Test 2) after testing compounds at different concentrations based on the IC₅₀ values of each compound.

Combination Index values computed from Compusyn Software for EGI-1 cells:

| **Olaparib + Ex 1** | **EC₅₀** | **EC₇₅** | **EC₉₀** | **EC₉₅** |
|---|---|---|---|---|
| **Test 1** | 0.645 | 0.469 | 0.348 | 0.287 |
| **Test 2** | 0.677 | 0.512 | 0.396 | 0.336 |

### Example 2) HPAF-II Cells (Test 1)

| | | | | | | | |
|---|---|---|---|---|---|---|---|
| **Olaparib** | **Dose** | **0** | **9.7975** | **19.595** | **39.19** | **78.38** | **156.76** |
| **IC₅₀ = 39.19 µM** | **Colony count** | 718 | 522 | 498 | 381 | 313 | 237 |
| | | 621 | 507 | 471 | 427 | 244 | 206 |
| **Ex 1** | **Dose** | **0** | **0.001929** | **0.003838** | **0.007677** | **0.015354** | **0.030707** |
| **IC₅₀ = 0.0077µM** | **Colony count** | 728 661 | 564 493 | 499 416 | 387 453 | 362 345 | 260 201 |
| **Olaparib+Ex 1** | **Colony count** | 624 | 404 | 354 | 271 | 138 | 137 |
| | | 692 | 419 | 358 | 343 | 161 | 209 |

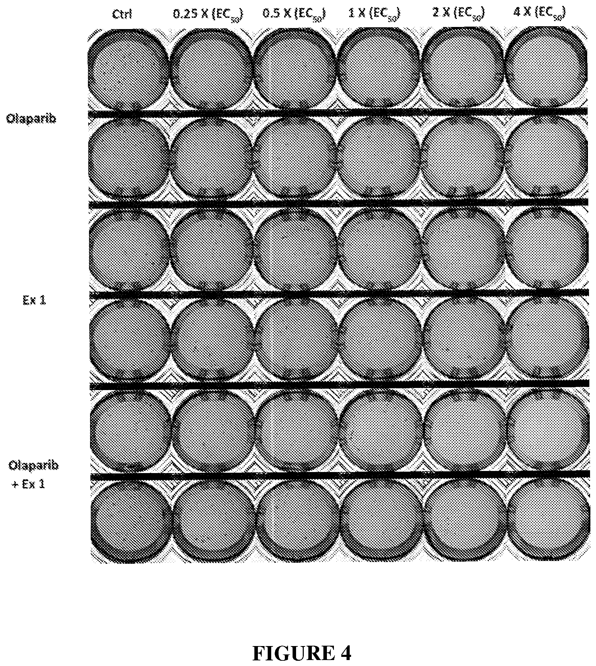

Figure 4 illustrates of a dose-response assay plate containing HPAF-II cells (Test 1) after testing compounds at different concentrations based on the IC₅₀ values of each compound. HPAF-II is a cell line established from pancreatic ductal adenocarcinoma (pancreatic cancer).

### HPAF-II Cells (Test 2)

| | | | | | | | |
|---|---|---|---|---|---|---|---|
| **Olaparib** | **Dose** | **0** | **9.7975** | **19.595** | **39.19** | **78.38** | **156.76** |
| **IC₅₀ = 39.19 µM** | **Colony count** | 845 | 550 | 443 | 278 | 239 | 142 |
| | | 696 | 426 | 375 | 248 | 172 | 92 |
| **Ex 1** | **Dose** | **0** | **0.001929** | **0.003838** | **0.007677** | **0.015354** | **0.030707** |
| **IC₅₀ = 0.0077 µM** | **Colony count** | 619 | 550 | 415 | 296 | 200 | 127 |
| | | 673 | 489 | 426 | 296 | 150 | 105 |
| **Olaparib+Ex 1** | **Colony count** | 610 | 330 | 197 | 126 | 54 | 62 |
| | | 580 | 272 | 184 | 109 | 59 | 66 |

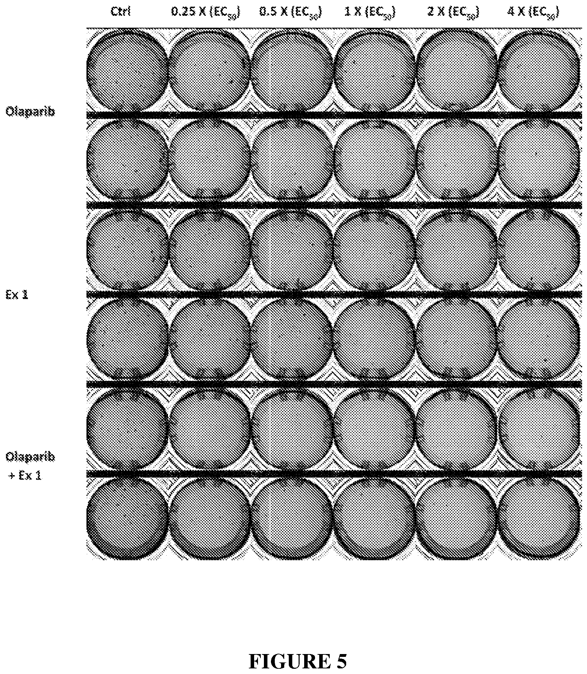

Figure 5 illustrates of a dose-response assay plate containing HPAF-II cells (Test 2) after testing compounds at different concentrations based on the IC₅₀ values of each compound.

Combination Index values computed from Compusyn Software for HPAF-II cells:

| **Olaparib + Ex 1** | **EC₅₀** | **EC₇₅** | **EC₉₀** | **EC₉₅** |
|---|---|---|---|---|
| **Test 1** | **0.729** | **0.652** | **0.593** | **0.562** |
| **Test 2** | **0.661** | **0.712** | **0.778** | **0.832** |

### Example 3) MCAS Cells (Test 1)

| | | | | | | | |
|---|---|---|---|---|---|---|---|
| **Olaparib** | **Dose** | **0** | **0.99475** | **1.9895** | **3.979** | **7.958** | **15.916** |
| **IC₅₀ = 3.979 µM** | **Colony count** | 768 | 429 | 363 | 273 | 188 | 112. |
| | | 634 | 425 | 402 | 227 | **141** | 97 |
| **Ex 1** | **Dose** | **0** | **0.07175** | **0.1435** | **0.287** | **0.574** | **1.148** |
| **IC₅₀ = 0.287 µM** | **Colony count** | 660 | 598 | 462 | 301 | 252 | 147 |
| | | 689 | 557 | 445 | 328 | 273 | 151 |
| **Olaparib+Ex 1** | **Colony count** | 567 | 302 | 155 | 73 | 46 | 134 |
| | | 579 | 271 | 173 | 100 | 45 | 44 |

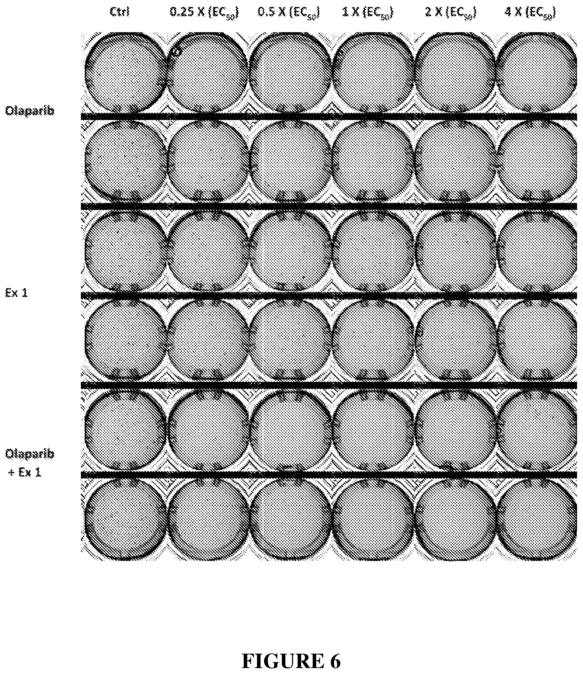

Figure 6 illustrates of a dose-response assay plate containing MCAS cells (Test 1) after testing compounds at different concentrations based on the IC₅₀ values of each compound. MCAS is a cell line established from ovarian mucinous cystadenocarcinoma (ovarian cancer).

### MCAS Cells (Test 2)

| | | | | | | | |
|---|---|---|---|---|---|---|---|
| **Olaparib** | **Dose** | **0** | **0.99475** | **1.9895** | **3.979** | **7.958** | **15.916** |
| **IC₅₀ = 3.979 µM** | **Colony count** | 1280 | 951 | 741 | 393 | 155 | 85 |
| | | 1366 | 929 | 668 | 359 | 123 | 82 |
| **Ex 1** | **Dose** | **0** | **0.07175** | **0.1435** | **0.287** | **0.574** | **1.148** |
| **IC₅₀ = 0.287 µM** | **Colony count** | 1076 | 976 | 744 | 694 | 644 | 419 |
| | | 1037 | 921 | 875 | 706 | 588 | 326 |
| **Olaparib+Ex 1** | **Colony count** | 1223 | 791 | 483 | 148 | 58 | 43 |
| | | 1164 | 741 | 452 | 187 | 67 | 46 |

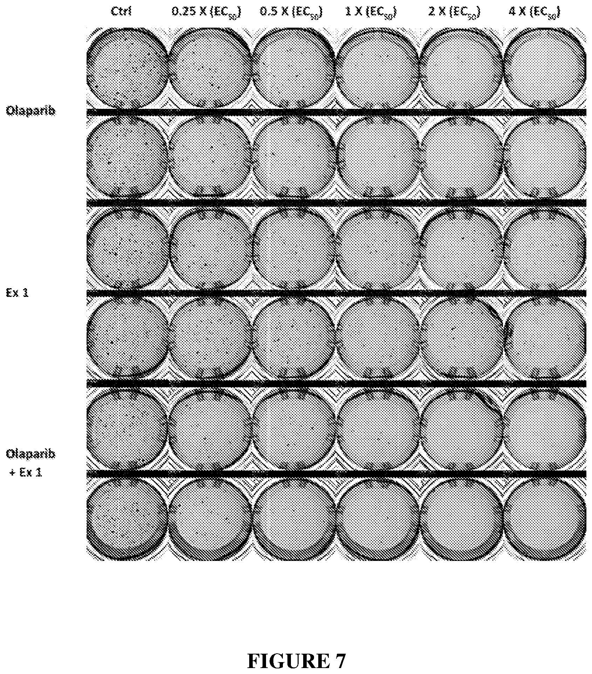

Figure 7 illustrates a dose-response assay plate containing MCAS cells (Test 2) after testing compounds at different concentrations based on the IC₅₀ values of each compound.

Combination Index values computed from Compusyn Software for MCAS cells:

| **Olaparib** + **Ex 1** | **EC₅₀** | **EC₇₅** | **EC₉₀** | **EC₉₅** |
|---|---|---|---|---|
| **Test 1** | 0.583 | 0.534 | 0.494 | 0.471 |
| **Test 2** | 0.838 | 0.7494 | 0.681 | 0.643 |

### Example 4) CFPAC-1 Cells (Test 1)

| | | | | | | | |
|---|---|---|---|---|---|---|---|
| **Olaparib** | **Dose** | **0** | **1.5625** | **3.125** | **6.25** | **12.5** | **25** |
| **IC₅₀ = 6.25 µM** | **Colony count** | 592 | 441 | 314 | 259 | 194 | 80 |
| | | 494 | 429 | 309 | 289 | 196 | 97 |
| **Ex 1** | **Dose** | **0** | **0.00675** | **0.0135** | **0.027** | **0.054** | **0.108** |
| **IC₅₀ = 0.027 µM** | **Colony count** | 549 | 441 | 445 | 386 | 346 | 315 |
| | | 542 | 464 | 505 | 412 | 322 | 320 |
| **Olaparib+Ex 1** | **Colony count** | 350 | 289 | 239 | 140 | 88 | 65 |
| | | 382 | 254 | 181 | 125 | 101 | 40 |

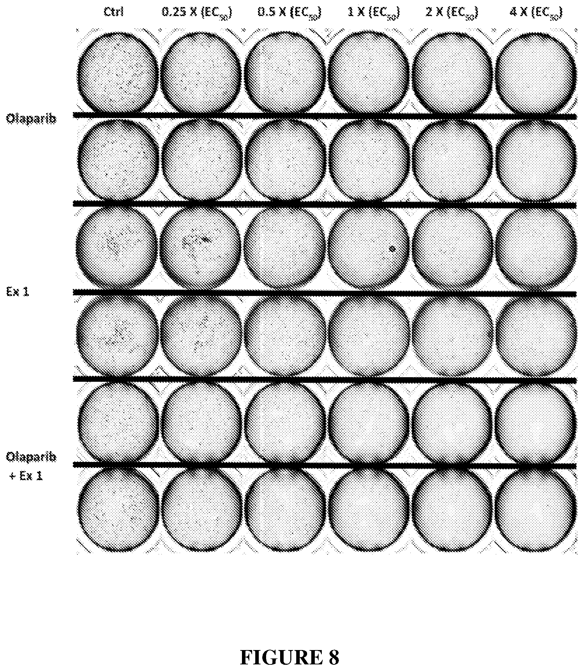

Figure 8 illustrates a dose-response assay plate containing CFPAC-1 cells (Test 1) after testing compounds at different concentrations based on the IC₅₀ values of each compound. CFPAC-1 is a cell line established from cystic fibrosis and pancreatic ductal adenocarcinoma.

### CFPAC-1 Cells (Test 2)

| | | | | | | | |
|---|---|---|---|---|---|---|---|
| **Olaparib** | **Dose** | **0** | **1.5625** | **3.125** | **6.25** | **12.5** | **25** |
| **IC₅₀ =6.25 µM** | **Colony count** | 714 | 586 | 439 | 328 | 254 | 116 |
| | | 670 | 545 | 442 | 371 | 255 | 111 |
| **Ex 1** | **Dose** | **0** | **0.00675** | **0.0135** | **0.027** | **0.054** | **0.108** |
| **IC₅₀ = 0.027µM** | **Colony count** | 663 | 676 | 693 | 649 | 581 | 536 |
| | | 670 | 624 | 666 | 563 | 588 | 527 |
| **Olaparib+Ex 1** | **Colony count** | 668 | 480 | 283 | 252 | 152 | 61 |
| | | 678 | 461 | 343 | 212 | 137 | 51 |

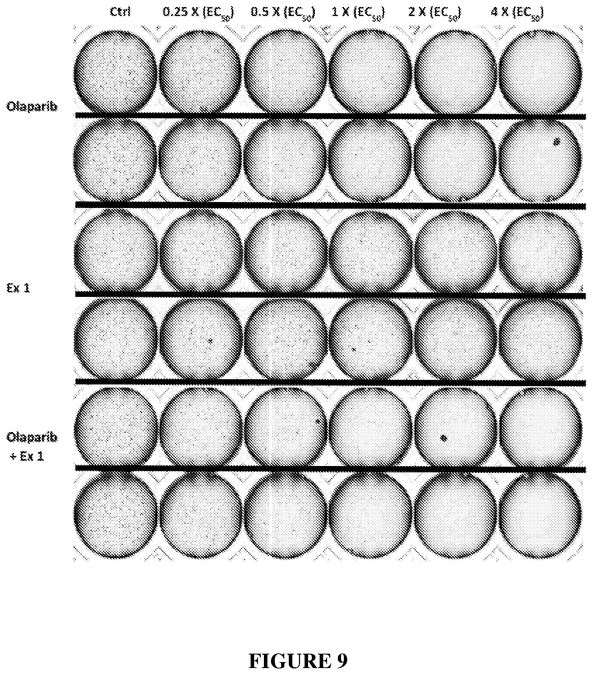

Figure 9 illustrates a dose-response assay plate containing CFPAC-1 cells (Test 2) after testing compounds at different concentrations based on the IC₅₀ values of each compound.

Combination Index values computed from Compusyn Software for CFPAC-1 cells:

| **Olaparib + Ex 1** | **ECso** | **EC₇₅** | **EC₉₀** | **EC₉₅** |
|---|---|---|---|---|
| **Test 1** | 0.840 | 0.762 | 0.727 | 0.713 |
| **Test 2** | 0.567 | 0.540 | 0.514 | 0.498 |

### Example 5) TFK-1 Cells (Test 1)

| | | | | | | | |
|---|---|---|---|---|---|---|---|
| **Olaparib** | **Dose** | **0** | **4.5** | **9** | **18** | **36** | **72** |
| **IC₅₀ = 18 µM** | **Colony count** | 875 | 759 | 674 | 381 | 270 | 96 |
| | | 894 | 668 | 531 | 437 | 204 | 75 |
| **Ex 1** | **Dose** | **0** | **3** | **6** | **23** | **24** | **48** |
| **IC₅₀ = 12 µM** | **Colony count** | 761 | 921 | 805 | 557 | 402 | 239 |
| | | 915 | 950 | 700 | 581 | 384 | 198 |
| **Olaparib+Ex 1** | **Colony count** | 821 | 584 | 437 | 178 | 68 | 10 |
| | | 855 | 599 | 431 | 205 | 78 | 11 |

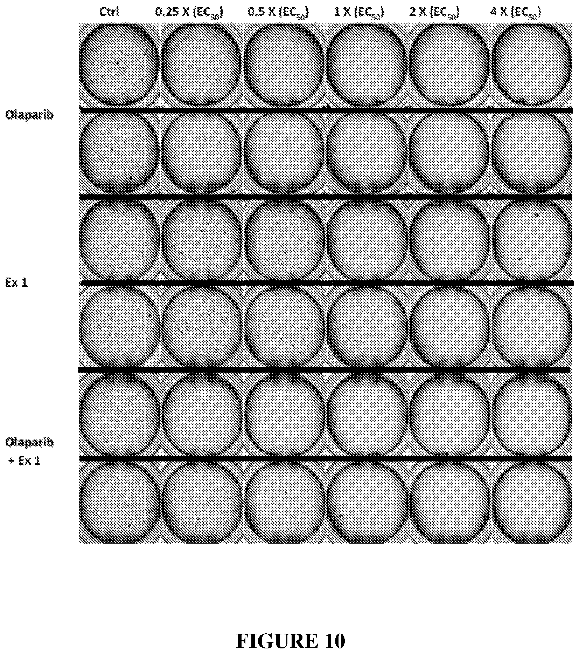

Figure 10 illustrates a dose-response assay plate containing TFK-1 cells (Test 1) after testing compounds at different concentrations based on the IC₅₀ values of each compound. TFK-1 is a cell line established from cholangiocarcinoma (bile duct adenocarcinoma).

### TFK-1 Cells (Test 2)

| | | | | | | | |
|---|---|---|---|---|---|---|---|
| **Olaparib** | **Dose** | **0** | **4.5** | **9** | **18** | **36** | **72** |
| **IC₅₀ = 18 µM** | **Colony count** | 1009 | 737 | 698 | 447 | 267 | 148 |
| | | 982 | 676 | 621 | 437 | 213 | 75 |
| **Ex 1** | **Dose** | **0** | **3** | **6** | **23** | **24** | **48** |
| **IC₅₀ = 12 µM** | **Colony count** | 982 | 916 | 770 | 567 | 336 | 101 |
| | | 943 | 840 | 818 | 559 | 249 | 148 |
| **Olaparib+Ex 1** | **Colony count** | 928 | 516 | 346 | 129 | 50 | 9 |
| | | 858 | 591 | 393 | 160 | 41 | 8 |

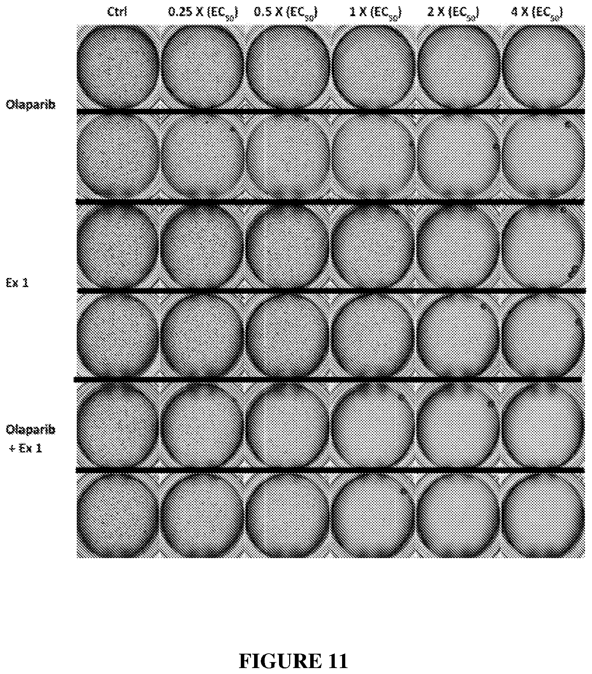

Figure 11 illustrates a dose-response assay plate containing TFK-1 cells (Test 2) after testing compounds at different concentrations based on the IC₅₀ values of each compound.

Combination Index values computed from Compusyn Software for TFK-1 cells:

| **Olaparib + Ex 1** | **EC₅₀** | **EC₇₅** | **EC₉₀** | **EC₉₅** |
|---|---|---|---|---|
| **Test 1** | 0.817 | 0.663 | 0.540 | 0.470 |
| **Test 2** | 0.835 | 0.634 | 0.491 | 0.417 |

### Example 6) PA-TU-8988T Cells (Test 1)

| | | | | | | | |
|---|---|---|---|---|---|---|---|
| **Olaparib** | **Dose** | **0** | **0.2525** | **0.505** | **1.01** | **2.02** | **4.04** |
| **IC₅₀ =1.01 µM** | **Colony count** | 127 | 537 | 425 | 398 | 411 | 362 |
| | | 570 | 426 | 331 | 327 | 325 | 307 |
| **Ex 1** | **Dose** | **0** | **0.275** | **0.55** | **1.1** | **2.2** | **4.4** |
| **IC₅₀ = 1.1 µM** | **Colony count** | 642 | 420 | 343 | 215 | 250 | 212 |
| | | 590 | 494 | 488 | 393 | 311 | 296 |
| **Olaparib+Ex 1** | **Colony count** | 616 | 331 | 219 | 206 | 110 | 63 |
| | | 593 | 262. | 222 | 100 | 107 | 68 |

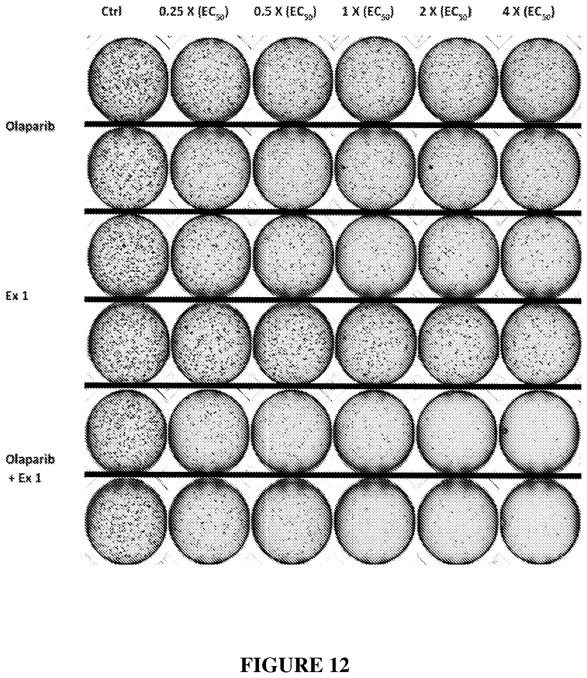

Figure 12 illustrates a dose-response assay plate containing PA-TU-8988T cells (Test 1) after testing compounds at different concentrations based on the IC₅₀ values of each compound. MCAS is a cell line established from pancreatic adenocarcinoma (pancreatic cancer).

### PA-TU-8988T Cells (Test 2)

| | | | | | | | |
|---|---|---|---|---|---|---|---|
| **Olaparib** | **Dose** | **0** | **0.2525** | **0.505** | **1.01** | **2.02** | **4.04** |
| **IC₅₀ =1.01 µM** | **Colony count** | 255 | 222 | 180 | 146 | 136 | 180 |
| | | 243 | 170 | 155 | 150 | 143 | 125 |
| **Ex 1** | **Dose** | **0** | **0.275** | **0.55** | **1.1** | **2.2** | **4.4** |
| **IC₅₀ = 1.1 µM** | **Colony count** | 290 | 200 | 143 | 156 | 155 | 125 |
| | | 297 | 237 | 227 | 209 | 215 | 186 |
| **Olaparib+Ex 1** | **Colony count** | 224 | 182 | 143 | 120 | 84 | 90 |
| | | 232 | 142 | 122 | 109 | 88 | 94 |

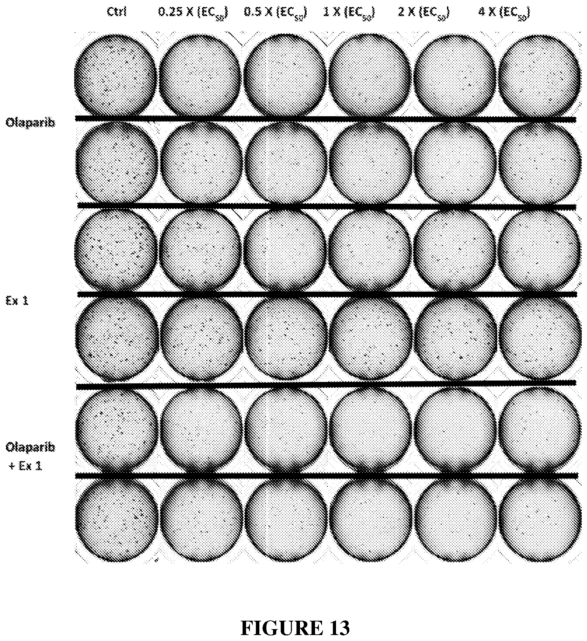

Figure 13 illustrates a dose-response assay plate containing PA-TU-8988T cells (Test 2) after testing compounds at different concentrations based on the IC₅₀ values of each compound.

Combination Index values computed from Compusyn Software for PA-TU-8988T cells:

| **Olaparib + Ex 1** | **EC₅₀** | **EC₇₅** | **EC₉₀** | **EC₉₅** |
|---|---|---|---|---|
| **Test 1** | 0.257 | 0.114 | 0.057 | 0.037 |
| **Test 2** | 0.468 | 0.157 | 0.063 | 0.035 |

### Example 7) Inhibition of Wnt signalling reduces Expression of DNA repair genes

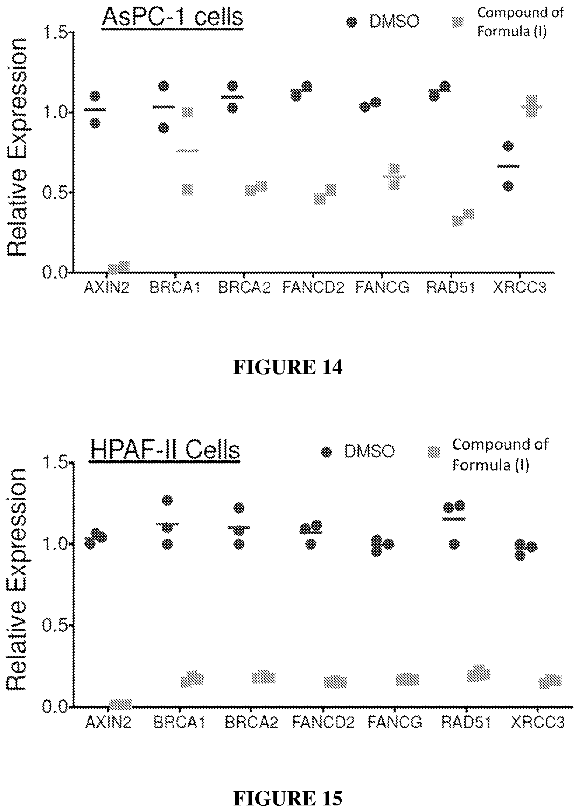

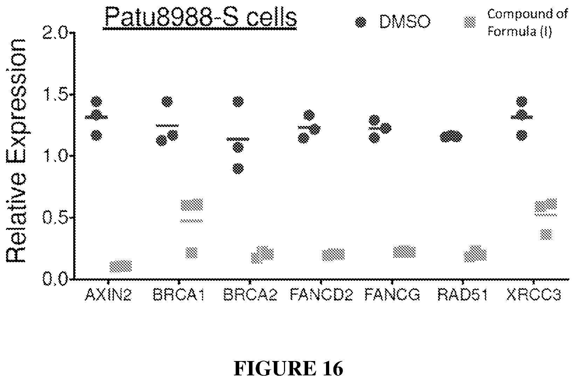

Inhibition of Wnt signalling reduces expression of DNA repair genes: HPAF-II, AsPC-1 or PaTu899S cells were treated with either DMSO or 100 nM compound of Formula (I) for 48 hr. The expression of the indicated genes was measured by qRT-PCR. Figures 14-16 illustrates the results.

### Example 8) Combination synergise to prevent growth of HPAF-II xenografts

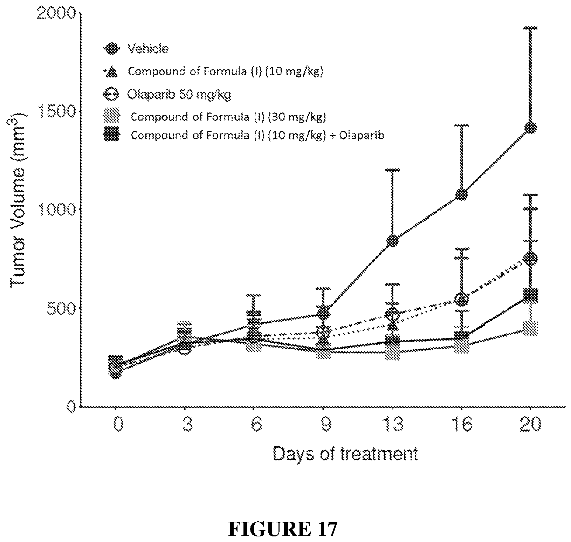

The combination of compound of Formula (I) and PARP inhibitor (Olaparib) synergise to prevent growth of HPAF-II xenografts. NSG mice with established HPAF-II subcutaneous xenografts were randomised into five groups. Mice were gavaged daily with indicated doses of compound of Formula (I), Olaparib (50 mg/kg) or a combination. Treatment was initiated after 10 days of injecting HPAF-II cells and the treatment was continued for 21 days. The tumor volumes as measured at the start of the treatement and during the course of treatment are shown in Figure 17. Data represent n= 10-12 tumors/group and represent Mean ± SD.

### Conclusion

Six different cell lines associated with pancreatic, ovarian and cholangio-carcinomas were treated with compound of formula (I) and Olaparib. The effect of these drug combinations was tested using an *in vitro* soft agar colony formation assay. In all cell lines, the combination index values were obtained using the Compusyn software. The combination index values obtained via independent experiments for all cell lines were found to be less than 0.9, indicating that the combination of compound of formula (I) and Olaparib is synergistic.

Mice bearing subcutaneous HPAF-II subcutaneous xenografts were treated with compound of Formula (I), PARP inhibitor or a combination of both. Treatment with a low dose of compound of Formula (I) and Olaparib inhibited the growth of the xenografts more effectively compared to either compounds alone.

Three pancreatic cancer cell lines were treated with compound of Formula (I). The expression of DNA repair genes was measured by q-RT-PCR. In all the three cell lines tested, treatment with compound of Formula (I) inhibited the expression of DNA repair genes BRCA1, BRCA2, FANCD2, FANCG, RAD51 and XRCC3.

| | | | | | | | |
|---|---|---|---|---|---|---|---|
| **Olaparib** | **Dose** | **0** | **0.2525** | **0.505** | **1.01** | **2.02** | **4.04** |
| **IC₅₀ =1.01 µM** | **Colony count** | **255** | **222** | **180** | **146** | **136** | **180** |
| | | **243** | **170** | **155** | **150** | **143** | **125** |
| **Ex 1** | **Dose** | **0** | **0.275** | **0.55** | **1.1** | **2.2** | **4.4** |
| **IC₅₀ = 1.1 µM** | **Colony count** | **290** | **200** | **143** | **156** | **155** | **125** |
| | | **297** | **237** | **227** | **209** | **215** | **186** |
| **Olaparib+Ex 1** | **Colony count** | **224** | **182** | **143** | **120** | **84** | **90** |
| | | **232** | **142** | **122** | **109** | **88** | **94** |

Figure 13 illustrates a dose-response assay plate containing PA-TU-8988T cells (Test 2) after testing compounds at different concentrations based on the IC₅₀ values of each compound.

Combination Index values computed from Compusyn Software for PA-TU-8988T cells:

| **Olaparib + Ex 1** | **EC₅₀** | **EC₇₅** | **EC₉₀** | **EC₉₅** |
|---|---|---|---|---|
| **Test 1** | 0.257 | 0.114 | 0.057 | 0.037 |
| **Test 2** | 0.468 | 0.157 | 0.063 | 0.035 |

### Example 7) Inhibition of Wnt signalling reduces Expression of DNA repair genes

Inhibition of Wnt signalling reduces expression of DNA repair genes: HPAF-II, AsPC-1 or PaTu899S cells were treated with either DMSO or 100 nM compound of Formula (I) for 48 hr. The expression of the indicated genes was measured by qRT-PCR. Figures 14-16 illustrates the results.

### Example 8) Combination synergise to prevent growth of HPAF-II xenografts

The combination of compound of Formula (I) and PARP inhibitor (Olaparib) synergise to prevent growth of HPAF-II xenografts. NSG mice with established HPAF-II subcutaneous xenografts were randomised into five groups. Mice were gavaged daily with indicated doses of compound of Formula (I), Olaparib (50 mg/kg) or a combination. Treatment was initiated after 10 days of injecting HPAF-II cells and the treatment was continued for 21 days. The tumor volumes as measured at the start of the treatement and during the course of treatment are shown in Figure 17. Data represent n= 10-12 tumors/group and represent Mean ± SD.

### Conclusion

Six different cell lines associated with pancreatic, ovarian and cholangio-carcinomas were treated with compound of formula (I) and Olaparib. The effect of these drug combinations was tested using an *in vitro* soft agar colony formation assay. In all cell lines, the combination index values were obtained using the Compusyn software. The combination index values obtained via independent experiments for all cell lines were found to be less than 0.9, indicating that the combination of compound of formula (I) and Olaparib is synergistic.

Mice bearing subcutaneous HPAF-II subcutaneous xenografts were treated with compound of Formula (I), PARP inhibitor or a combination of both. Treatment with a low dose of compound of Formula (I) and Olaparib inhibited the growth of the xenografts more effectively compared to either compounds alone.

Three pancreatic cancer cell lines were treated with compound of Formula (I). The expression of DNA repair genes was measured by q-RT-PCR. In all the three cell lines tested, treatment with compound of Formula (I) inhibited the expression of DNA repair genes BRCA1, BRCA2, FANCD2, FANCG, RAD51 and XRCC3.

## Claims

1. A pharmaceutical combination for use in treating pancreatic cancer, ovarian cancer, or cholangio-carcinomas comprising: (a) a compound of formula (I) or a pharmaceutically acceptable salt, or solvate thereof and (b) Olaparib.

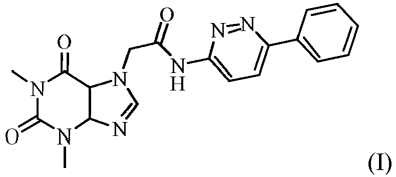

2. The pharmaceutical combination for use according to claim 1, wherein (a) the compound of formula (I) and (b) Olaparib are for administration simultaneously or sequentially in any order.

3. A pharmaceutical composition comprising (a) a compound of formula (I) or a pharmaceutically acceptable salt or solvate thereof and (b) Olaparib.

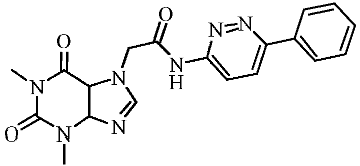

4. The pharmaceutical combination for use according to claim 1 or 2, wherein the compound of Formula (I) is administered daily at 0.05 mg/kg to 20 mg/kg.

5. The pharmaceutical combination for use according to claims 1 or 2, wherein compound of Formula (I) is I.V. administered on day 1 and 8 of a 21 day cycle, at 2 mg/kg to 24 mg/kg; and
wherein Olaparib is orally administered daily.

6. The pharmaceutical combination for use according to any one of claims 1, 2, 4, and 5, wherein compound of formula (I) is administered at 50 mg to 2000 mg per dose, and Olaparib is administered at 100 mg to 400 mg per dose.

7. The pharmaceutical combination according to claim 3, wherein the pharmaceutical composition comprises compound of Formula (I) and Olaparib each independently for oral administration at 0.02 mg to 50 mg.

8. The pharmaceutical combination according to claim 3, wherein the pharmaceutical composition comprises compound of Formula (I) at 50 mg to 2000 mg, and Olaparib at 100 mg to 400 mg.

## Patentansprüche

1. Pharmazeutische Kombination zur Behandlung von Bauchspeicheldrüsenkrebs, Eierstockkrebs oder Cholangiokarzinomen, umfassend: (a) eine Verbindung der Formel (I) oder ein pharmazeutisch unbedenkliches Salz oder Solvat davon 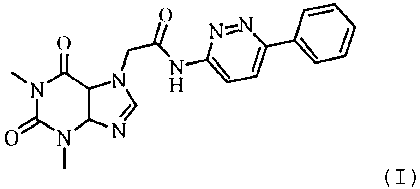 und (b) Olaparib.

2. Pharmazeutische Kombination zur Verwendung nach Anspruch 1, wobei (a) die Verbindung der Formel (I) und (b) Olaparib zur gleichzeitigen oder aufeinanderfolgende Verabreichung in beliebiger Reihenfolge bestimmt sind.

3. Pharmazeutische Zusammensetzung, umfassend (a) eine Verbindung der Formel (I) oder ein pharmazeutisch unbedenkliches Salz oder Solvat davon 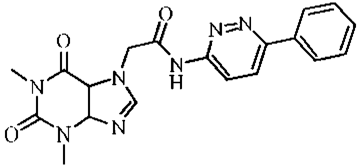 und (b) Olaparib.

4. Pharmazeutische Kombination zur Verwendung nach Anspruch 1 oder 2, wobei die Verbindung der Formel (I) täglich in einer Menge von 0,05 mg/kg bis 20 mg/kg verabreicht wird.

5. Pharmazeutische Kombination zur Verwendung nach Anspruch 1 oder 2, wobei die Verbindung der Formel (I) an Tag 1 und 8 eines 21-Tage-Zyklus in einer Menge von 2 mg/kg bis 24 mg/kg i.v. verabreicht wird; und
wobei Olaparib täglich oral verabreicht wird.

6. Pharmazeutische Kombination zur Verwendung nach einem der Ansprüche 1, 2, 4 und 5, wobei die Verbindung der Formel (I) in einer Menge von 50 mg bis 2000 mg pro Dosis und Olaparib in einer Menge von 100 mg bis 400 mg pro Dosis verabreicht wird.

7. Pharmazeutische Kombination nach Anspruch 3, wobei die pharmazeutische Zusammensetzung die Verbindung der Formel (I) und Olaparib jeweils unabhängig zur oralen Verabreichung in einer Menge von 0,02 mg bis 50 mg umfasst.

8. Pharmazeutische Kombination nach Anspruch 3, wobei die pharmazeutische Zusammensetzung die Verbindung der Formel (I) in einer Menge von 50 mg bis 2000 mg und Olaparib in einer Menge von 100 mg bis 400 mg umfasst.

## Revendications

1. Combinaison pharmaceutique destinée à être utilisée dans le traitement du cancer du pancréas, du cancer de l'ovaire ou de cholangio-carcinomes comprenant : (a) un composé de formule (I) ou un sel pharmaceutiquement acceptable, ou un solvate de celui-ci 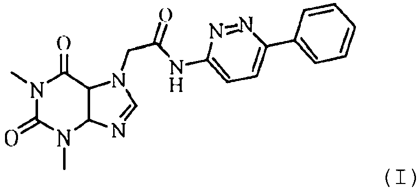 et b) de l'Olaparib.

2. Combinaison pharmaceutique destinée à être utilisée selon la revendication 1, dans laquelle (a) le composé de formule (I) et (b) l'Olaparib sont destinés à être administrés simultanément ou séquentiellement dans un ordre quelconque.

3. Composition pharmaceutique comprenant (a) un composé de formule (I) ou un sel pharmaceutiquement acceptable ou solvate de celui-ci 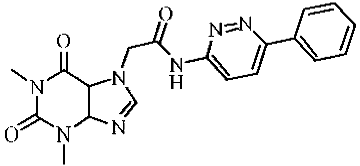 et b) de l'Olaparib.

4. Combinaison pharmaceutique pour utilisation selon la revendication 1 ou 2, dans laquelle le composé de Formule (I) est administré quotidiennement à raison de 0,05 mg/kg à 20 mg/kg.

5. Combinaison pharmaceutique pour utilisation selon la revendication 1 ou 2, dans laquelle le composé de Formule (I) est administré par voie intraveineuse aux jours 1 et 8 d'un cycle de 21 jours, à raison de 2 mg/kg à 24 mg/kg ; et
dans laquelle l'Olaparib est administré quotidiennement par voie orale.

6. Combinaison pharmaceutique pour utilisation selon l'une quelconque des revendications 1, 2, 4 et 5, dans laquelle le composé de formule (I) est administré à raison de 50 mg à 2 000 mg par dose, et l'Olaparib est administré à raison de 100 mg à 400 mg par dose.

7. Combinaison pharmaceutique selon la revendication 3, dans laquelle la composition pharmaceutique comprend un composé de Formule (I) et de l'Olaparib chacun indépendamment pour une administration orale à raison de 0,02 mg à 50 mg.

8. Combinaison pharmaceutique selon la revendication 3, dans laquelle la composition pharmaceutique comprend un composé de Formule (I) à raison de 50 mg à 2 000 mg, et de l'Olaparib à raison de 100 mg à 400 mg.
